# FASCICULE DE BREVET EUROPEEN

(11) **EP 1 163 350 B1**
(45) Date de publication et mention de la délivrance du brevet: **09.11.2005**
(21) Numéro de dépôt: 00912722.6
(22) Date de dépôt: 22.03.2000
(51) Int. Cl.: C12N 15/80, C12N 15/65

(54) **POLYNUCLEOTIDES POUR LA MUTAGENESE INSERTIONNELLE CHEZ LE CHAMPIGNON COMPRENANT UN GENE FONCTIONNEL DANS (MAGNAPORTHE) ET UN TRANSPOSON (IMPALA)**
POLYNUKLEOTIDE ZUR MUTAGENESE IN PILZEN, DIE EIN FUNKTIONELLES MAGNAPORTHE GEN UND EIN IMPALA TRANSPOSON ENTHALTEN
POLYNUCLEOTIDES FOR MUTAGENESIS IN FUNGUS COMPRISING A FUNCTIONAL GENE IN MAGNAPORTHE AND AN IMPALA TRANPOSON

(30) Priorité: 22.03.1999 FR 9903701
(43) Date de publication de la demande: 19.12.2001
(73) Titulaire: Bayer CropScience S.A., 69009 Lyon (FR)
(72) Inventeur: GROSJEAN-COURNOYER, Marie-Claire, 69300 Caluire (FR); VILLALBA, François, F-69005 Lyon (FR); LEBRUN, Marc-Henri, F-69002 Lyon (FR); DABOUSSI, Marie-Josée, F-91440 Bures-sur-Yvette (FR)
(86) Numéro de dépôt international: PCT/FR2000/000713
(87) Numéro de publication internationale: WO 2000/056902

(56) Documents cités:
- BROWN J S ET AL: "Insertional mutagenesis of pathogenic fungi." CURR OPIN MICROBIOL, (1998 AUG) 1 (4) 390-4. REF: 44, XP000856194
- HUA-VAN, A. (1) ET AL: "Three highly divergent subfamilies of the impala transposable element coexist in the genome of the fungus Fusarium oxysporum." MOLECULAR AND GENERAL GENETICS, (SEPT., 1998) VOL. 259, NO. 4, PP. 354-362., XP002124588
- LANGIN, THIERRY (1) ET AL: "The transposable element impala, a fungal member of the Tc1-mariner superfamily." MOLECULAR & GENERAL GENETICS, (1995) VOL. 246, NO. 1, PP. 19-28., XP002124589
- KACHROO, PRADEEP ET AL: "Organisation and molecular analysis of repeated DNA sequences in the rice blast fungus Magnaporthe grisea." CURRENT GENETICS, (1997) VOL. 31, NO. 4, PP. 361-369., XP000856212
- FARMAN, MARK L. ET AL: "The Magnaporthe grisea DNA fingerprinting probe MGR586 contains the 3' end of an inverted repeat transposon." MOLECULAR & GENERAL GENETICS, (1996) VOL. 251, NO. 6, PP. 675-681., XP002124590
- KACHROO, P. ET AL: "Pot2, an inverted repeat transposon from the rice blast fungus Magnaporthe grisea." MOLECULAR & GENERAL GENETICS, (1994) VOL. 245, NO. 3, PP. 339-348., XP002124591
- DURAND, NATHALIE ET AL: "Transformation of Penicillium roqueforti to phleomycin- and to hygromycin B-resistance" CURR. GENET. (1991), 19(2), 149-53,1991, XP000856213
- DABOUSSI, M. J. ET AL: "Transposable elements in the fungal plant pathogen Fusarium oxysporum." GENETICA (DORDRECHT), (1994) VOL. 93, NO. 1-3, PP. 49-59., XP000856193
- DOBINSON K.F. ET AL: "The ebb and flow of a fungal genome." TRENDS IN MICROBIOLOGY, (1993) 1/9 (348-352)., XP000856204
- T. LANGIN ET AL.: "Influence of biological parameters and gene transfer technique on transformation of Fusarium oxysporum." CURRENT GENETICS, vol. 17, no. 4, 1990, pages 313-319, XP000856211 cité dans la demande

### LISTE DE SEQUENCES

<110> Aventis CropScience SA
<120> Polynucléotides pour la mutagenèse insertionnelle chez le champignon comprenant un gène fonctionnel dans Magnaporthe et un transposon Impala
<130> PH 99013 G1

<140> PCT/FROO/00713
<141> 2000-03-22

<150> 9903701
<151> 1999-03-22

<160> 8
<170> PatentIn Ver. 2.1

<210> 1
<211> 20
<212> ADN
<213> Séquence artificielle

<220>
<223> Description de la séquence artificielle: primer SPE5

<400> 1
   agaacacaac cctgccacgg 20

<210> 2
<211> 20
<212> ADN
<213> Séquence artificielle

<220>
<223> Description de la séquence artificielle:primer SPE3

<400> 2
   tccgggccgt atgcacagag 20

<210> 3
<211> 18
<212> ADN
<213> séquence artificielle

<220>
<223> Description de la séquence artificielle:primer C1

<400> 3
   cgctgcgaat tcttcagt 18

<210> 4
<211> 18
<212> ADN
<213> Séquence artificielle
<220>
<223> Description de la séquence artificielle:primer niaX

<400> 4
   ctagacttag aacctcgg 18

<210> 5
<211> 20
<212> ADN
<213> séquence artificielle

<220>
<223> Description de la séquence artificielle:primer ImpE5'

<400> 5
   ggcattgaaa acgcggtccc 20

<210> 6
<211> 20
<212> ADN
<213> Séquence artificielle

<220>
<223> Description de la séquence artificielle:primer ImpE3'

<400> 6
   cagcagcaaa acagctgccc 20

<210> 7
<211> 20
<212> ADN
<213> Séquence artificielle

<220>
<223> Description de la séquence artificielle:primer hygl

<400> 7
   agcctgaact caccgcgacg 20

<210> 8
<211> 20
<212> ADN
<213> Séquence artificielle

<220>
<223> Description de la séquence artificielle:primer hyg4

<400> 8
   cgaccctgcg cccaagctgc 20

## Description

La présente invention concerne de nouveaux polynucléotides et l'utilisation de ces polynucléotides pour la mutagenèse insertionnelle et l'étiquetage de gènes chez les champignons. L'invention concerne également des collections de mutants de champignons obtenus par insertion aléatoire du transposon *Impala* de *Fusarium oxysporum* dans le génome de ces champignons. Ces collections de mutants représentent un outil génétique performant pour l'étude de la fonction des gènes chez les champignons.

Les transposons peuvent être définis comme des éléments génétiques mobiles capables de se déplacer entre deux séquences d'ADN. De par leur capacité à s'insérer dans des gènes (exons, introns, régions régulatrices), ils peuvent être la source de mutations. Ils participent, de ce fait, à l'évolution des génomes qu'ils parasitent. Les transposons ont été classifiés en deux groupes selon leur mode de propagation (Finnegan, 1989 ; Capy et al., 1998) :
- Les éléments de classe I (rétroéléments) transposent via un intermédiaire ARN rétrotranscrit en ADN par une transcriptase réverse. Cette classe se subdivise en rétroéléments ou rétrotransposons bordés ou non par des LTR (long terminal repeat). Parmi les rétroéléments à LTR se trouvent les éléments de la famille *gypsy* et de la famille *copia*. Ils possèdent des gènes homologues aux gènes *gag* et *pol* des rétrovirus et diffèrent par l'organisation des différents domaines fonctionnels du gène *pol*. De plus, la famille *gypsy* possède un gène homologue à *env* participant, chez les rétrovirus. à leur infectiosité. Parmi les rétroéléments sans LTR on distingue les LINEs possédant des gènes *gag, pol* et une séquence poly-A, ainsi que les SINEs, possédant également une queue poly-A mais dépourvus des séquences gag et *pol ;* ils sont supposés dériver des éléments LINEs antérieurs (Eickbush,1992 ; Okada and Hamada, 1997)
- Les éléments de classe II transposent via un mécanisme d'excision et réinsertion de la séquence d'ADN du transposon. Leur structure générale se compose de deux séquences répétées en orientation inverse (ITR) bordant une phase de lecture ouverte codant pour une transposase nécessaire à la transposition de l'élément. Ces éléments ont été regroupés en superfamilles, suivant les homologies des séquences de leur transposases et/ou des ITRs, dont celle des *Tel*/*mariner* (Doak et al., 1994), des *Foil*/*Pogo* (Capy et al., 1998 ; Smit et Riggs, 1996), des éléments hAT (Calvi et al., 1991), des éléments P (Kidwell, 1994) ou des éléments CACTA (Gierl 1996).

L'identification et l'étude des transposons de champignon représentent un très grand intérêt notamment en vue du développement d'outils de mutagenèse insertionnelle (Brown et al., Curr. Opin. Microbiol. 1:390-4, 1998) ainsi que pour l'étude du génome de ces champignons (Dobinson et al., Trends in Microbiology, 1:348-3652, 1993).

Différentes stratégies ont donc été mises en oeuvre pour identifier des transposons dans le génome des champignons. La première et la deuxième mettent à profit les connaissances qui dérivent des éléments caractérisés auparavant. Il s'agit de l'utilisation de sondes hétérologues utilisées dans des expériences d'hybridation de Southern ou des amplifications à l'aide d'oligonucléotides dérivés de domaines fortement conservés tels que celui de la transcriptase réverse des rétroéléments à LTR. La troisième stratégie consiste à caractériser des séquences d'ADN répétés. Dans ce cas, une hybridation différentielle entre l'ADN génomique et une sonde ribosomique est nécessaire. Des transposons de la famille Fot1 ont ainsi été identifiés dans le génome de *Magnaporthe grisea* (Kachroo et al., Current Genetics 31:361-369, 1997; Farman et al., Mol. Gen. Genetics 251:675-681 1996; Kachroo et al., Mol. Gen. Genetics 245:339-348,1994). La dernière méthode, à la différence des trois précédentes, permet d'identifier des éléments fonctionnels et actifs ; il s'agit du piège à transposon. Cette stratégie met en oeuvre l'inactivation d'un gène marqueur, dont la mutation engendrée par l'insertion de l'élément, peut être identifiée par un crible positif. Ainsi, le gène *am* (glutamate deshydrogénase) a permis de caractériser le rétroélément *Tad,* de type LINE, chez *Neurospora crassa*, à la suite de sa réinsertion dans ce gène (Kinsey et Helber, 1989). Le gène *niaD* (nitrate réductase) d'*Aspergillus nidulans* a également été utilisé pour piéger des transposons. En effet, une mutation inactivant ce gène confère une résistance au chlorate. Différents transposons ont ainsi été identifiés chez *Fusarium oxysporum* (Daboussi et al., Genetica 93:49-59, 1994) et chez *Aspergillus* (US 5,985,570). L'élément de classe II *Fotl* de *Fusarium oxysporum*, a été le premier transposon identifié grâce à l'inactivation du gène *niaD* (Daboussi *et al*., 1992). D'autre part, l'utilisation du gène *niaD* chez *Fusarium oxysporum* a permis de piéger le transposon *Impala* appartenant à la superfamille des éléments de type *Tcl*/*mariner* (Langin *et al*., 1995). Différentes sous-familles de transposons *Impala* ont été identifiés chez *Fusarium oxysporum* (Hua-Van et al., Mol. Gen. Genetics 259:354-362, 1998). La transposition de l'élément *Impala* a été étudié chez *Fusarium oxysporum*. Lorsque le transposon *Impala* est intégré dans le promoteur ou les introns d'un gène donné, il peut alors inactiver l'expression de ce gène. En revanche, après transposition du transposon *Impala*, le gène est réactivé ce qui constitue un contrôle positif de l'événement de transposition. Une telle stratégie d'identification de la transposition a été employée dans *Fusarium* avec un construit comprenant le transposon *Impala* intégré dans la séquence de régulation promotrice du gène de la nitrate reductase (*niaD*) *d'Aspergillus nidulans* (Hua-Van, 1998).

En utilisant le construit gène *niaD*/*Impala* du plasmide pNi 160 (Langin et al., 1995) dans d'autres champignons, et plus particulièrement *Magnaporthe grisea*, la transposition d'*Impala* n'a pu être mise en évidence, sinon à un taux extrêmement faible incompatible avec le développement d'un outil de mutagenèse insertionnelle. Ces observations suggèrent que le construit *niaD*/*Impala* du plasmide pNi 160 (Langin et al., 1995) et plus généralement que le transposon *Impala* lui-même ne sont pas fonctionnels dans d'autres champignons, et en particulier chez *M.grisea*.

Or, un tel outil permettant la création d'une collection de mutants d'insertion dans le génome de champignons et plus particulièrement de champignons filamenteux pathogènes est essentiel à l'étude de leur génome et à l'étude de la fonction de leurs gènes. L'analyse des fonctions des gènes de champignons est essentielle à la découverte de nouveaux composés antifongiques utiles pour le traitement des affections fongiques en santé humaine ou animale ou pour l'agriculture.

La présente invention concerne de nouveaux polynucléotides comprenant un gène marqueur fonctionnel dans *Magnaporthe grisea* inactivé par l'insertion d'un transposon *Impala.* Ces polynucléotides permettent de mettre en evidence la transposition de l'élément *Impala* dans les champignons avec un taux de transposition compatible avec le développement d'outils de mutagenèse insertionnelle. L'invention a donc également pour objet des méthodes de préparation de mutants de champignons par insertion d'un transposon *Impala* dans leur génome et des méthodes pour identifier un gène de champignon associé à un phénotype d'intérêt. Enfin, l'invention concerne des collections de mutants d'insertion de champignons et leurs utilisations.

### Description de l'invention

### Polynucléotides

La présente invention concerne donc un polynucléotide, en particulier un polynucléotide isolé ou purifié, comprenant un gène marqueur inactivé par l'insertion d'un transposon *Impala,* tel que ledit gène marqueur comprend dans le sens de la transcription une séquence de régulation promotrice fonctionnelle dans *Magnaporthe grisea* liée de manière fonctionnelle à la séquence codante dudit gène marqueur.

La transformation d'un champignon avec un polynucléotide selon l'invention et l'excision du transposon conduisent à l'expression du gène marqueur. La détection de l'expression du gène marqueur permet ainsi de suivre les évènements de transposition et de sélectionner les mutants d'insertion. La sélection des mutants peut être améliorée par le marquage du transposon par un second gène marqueur différent du premier. Ce second gène marqueur permet de suivre la réinsertion du transposon dans le génome du champignon. L'invention a donc également pour objet des polynucléotides tel que décrits ci-dessus dans lesquels le transposon *Impala* comprend un gène marqueur.

Les gènes marqueurs utilisés sont appropriés pour sélectionner les événements de transposition au moyen d'un crible simple. Tout gène marqueur dont l'expression dans un champignon peut être détectée par un crible phénotypique peut être utilisé dans la présente invention. Il est entendu que le terme "gène marqueur" désigne également des gènes chimères comprenant des éléments génétiques d'origines différentes. Les gènes marqueurs de la présente invention peuvent ainsi combiner une séquence promotrice de champignon et une séquence codante d'un gène marqueur ne provenant pas d'un champignon.

Par séquence de régulation promotrice fonctionnelle dans *Magnaporthe grisea* on entend selon l'invention tout polynucléotide permettant l'expression dans *Magnaporthe grisea* d'une séquence codante à laquelle il est lié de manière fonctionnelle. Les techniques permettant de déterminer si une séquence promotrice est fonctionnelle dans *Magnaporthe grisea* sont connues de l'homme du métier. Par exemple, *Magnaporthe* peut être transformé avec un polynucléotide comprenant dans le sens de la transcription une séquence promotrice potentielle et un gène rapporteur. Le suivi de l'expression du gène rapporteur dans le champignon transformé avec ce polynucléotide permet de déterminer si cette séquence promotrice est fonctionnelle dans *Magnaporthe*. Toute séquence promotrice peut ainsi être testée pour déterminer sa fonctionnalité dans *Magnaporthe grisea*. La séquence de régulation promotrice peut être une séquence de régulation promotrice d'un gène de *Magnaporthe grisea*, ou peut provenir d'un autre champignon, et plus particulièrement d'un autre champignon filamenteux. De manière avantageuse, la séquence de régulation promotrice fonctionnelle dans *Magnaporthe grisea* est constituée par la séquence de régulation promotrice d'un gène *nia* (nitrate reductase) ou *gpd* de champignon. De préférence, la séquence de régulation promotrice fonctionnelle dans *Magnaporthe grisea* est constituée par une séquence de régulation promotrice fonctionnelle dans *Magnaporthe* du gène *niaD* (Malardier et al., 1989) ou *gpdA* d'*Aspergillus nidulans* (Punt et al., 1990). Pour être fonctionnelle dans *Magnaporthe*, la séquence de régulation promotrice du gène *niaD d'Aspergillus nidulans* a une taille de préférence supérieure à 337 bp, à 0,4 kb, à 0,5 kb, à 0,6kb, à 0,7kb, à 0,8 kb, à 0,9 kb et plus préférentiellement supérieure ou égale à environ 1 kb. Dans un mode de réalisation préféré de l'invention, la séquence promotrice fonctionnelle dans *Magnaporthe grisea* est constitué d'un polynucléotide de 1.3 kb correspondant au fragment intergénique entre le gènes *niaD* et *niiA* d'*Aspergillus nidulans* (Genbank M58291; Johnstone et al., Gene 90:181-192, 1990; Punt et al., 1995). Il est entendu qu'une séquence de régulation fonctionnelle partielle, non fonctionnelle en tant que telle mais dont l'intégration aléatoire dans le génome de *Magnaporthe grisea* en aval d'un promoteur de *Magnaporthe grisea* permet l'expression du gène marqueur, n'est pas une séquence de régulation fonctionnelle dans *Magnaporthe grisea* selon la présente invention.

Selon un mode préférentiel de réalisation de l'invention, la séquence codante du gène marqueur est choisie parmi les séquences codantes d'un gène rapporteur dont l'expression est facilement mesurée, en particulier GUS (US 5 268 463, US 5 599 670) ou GFP (US 5 491 084, US 5 741 668), les séquences codantes pour un gène de tolérance à un antibiotique ou un herbicide, comme les gènes de résistance à l'hygromycine *(hph :* Punt *et al*., 1987), à la phléomycine (*ble* : Drocourt, 1990), à l'herbicide bialaphos *(Bar :* Pall et Brunelli, 1993), ou un gène de résistance aux sulfonylurées (Sweigard *et al*., 1997). Selon un autre mode préférentiel de réalisation de l'invention, le gène marqueur est choisie parmi les séquences de gènes codant pour des enzymes fonctionnelles dans les champignons. De manière avantageuse, le gène marqueur est le gène de la nitrate réductase. Lorsque l'on intègre le fragment selon l'invention dans un champignon nia-, la souche transformée avec cette construction conserve un phénotype mutant. L'apparition de colonies nia+ sur un milieu minimum (NaNO₃ comme seule source d'azote) révèle l'excision du transposon *Impala* permettant l'expression du gène *niaD*. Ces révertants nia+ peuvent être sélectionnés sur ce milieu grâce à leur phénotype dense et aérien différent du phénotype ras des colonies nia-. L'utilisation du gène *niaD* comme marqueur nécessite l'utilisation d'un champignon nia-. Les méthodes d'identification de champignons nia- sont bien connues de l'homme du métier. On citera notamment la méthode décrite par Daboussi et al. (1989).

Les polynucléotides de la présente invention comprennent un gène marqueur tel que décrit ci-dessus inactivé par l'insertion d'un transposon *Impala* (Langin *et al*., 1995; Hua-Van et al., 1998). Le transposon *Impala* comprend une phase ouverte de lecture codant pour une transposase fonctionnelle bordée de séquences répétées inversées (ITRs). Il s'insère au niveau d'un dinucléotide TA qui se retrouve dupliqué. L'excision d'*Impala* est imprécise et laisse le plus souvent une cicatrice de trois nucléotides, correspondant à l'extrémité gauche ou droite de l'élément, en plus du dinucléotide TA dupliqué lors de l'insertion. Le point d'insertion du transposon *Impala* dans le gène marqueur doit donc être choisi de manière à ce que suite à l'excision, la cicatrice résiduelle n'empêche pas l'expression du gène marqueur. De préférence, le transposon *Impala* est inséré dans la séquence promotrice ou dans un intron du gène marqueur. Plusieurs copies *d'Impala* ont été identifiées chez *Fusarium oxysporum* et la comparaison de leur séquence a permis de définir trois sous-familles possédant des ITRs de longueur et séquence variable (Hua-Van et al., 1998). Dans un mode de réalisation préféré, les polynucléotides de la présente invention comprennent un transposon *Impala 160.* L'élément *Impala 160* comporte 1280 pb, il est bordé par deux séquences répétées et inversées de 27 pb encadrant une phase de lecture ouverte codant pour une transposase de 340 acides aminés (Langin *et al*., 1995; Genbank S75106). Dans un mode de réalisation préféré, les polynucléotides de la présente invention comprennent le promoteur de 1.3 kb du gène *niaD d'Aspergillus nidulans* lié de manière fonctionnelle à la séquence codante du gène *niaD* d'*Aspergillus nidulans* et un transposon *Impala 160* inséré dans le promoteur du gène *niaD*. Dans un mode de réalisation particulièrement avantageux de l'invention, les polynucléotides de la présente invention comprennent le plasmide pNiL160. Ces construits sont utilisés pour transformer un champignon nia- et les mutants d'insertion résultant de la transposition de l'élément *Impala* sont sélectionnés pour leur phénotype nia+ sur un milieu minimum. Dans un autre mode de réalisation préféré, les polynucléotides de la présente invention comprennent le promoteur du gène *gpd* d'*Aspergillus nidulans* lié de manière fonctionnelle à la séquence codante du gène *hph* de résistance à l'hygromycine et un transposon *Impala 160* inséré dans le promoteur du gène *gpd*. Ces polynucléotides sont utilisés pour transformer un champignon et pour sélectionner les mutants d'insertion résistants à l'hygromycine résultant de la transposition de l'élément *Impala.*

Tout transposon *Impala* peut être utilisé dans les construits et les méthodes de la présente invention. Il est entendu que le terme "transposon *Impala"* désigne également des transposons *Impala* modifiés. Parmi ces modifications on citera notamment l'insertion d'un gène marqueur ou de séquences activatrices dans le transposon *Impala* ou encore l'inactivation de la transposase pour obtenir un transposon *Impala* défectif. La construction de ces transposons modifiés met en oeuvre des techniques classiques de biologie moléculaire bien connues de l'homme du métier.

Les polynucléotides de la présente invention sont préférentiellement utilisés pour l'obtention de mutants d'insertions de champignons. L'insertion du transposon *Impala* dans un gène conduit généralement à l'inactivation totale ou partielle de ce gène. L'utilisation d'un transposon *Impala* comprenant des séquences activatrices permet au contraire d'obtenir des mutants de sur-expression. Les modifications du transposons permettent ainsi la mise en oeuvre de différentes méthodes de mutagénèse insertionnelle (Bancroft et al., Mol. Gen.Genet. 233:449-461, 1992; Bancroft et Dean, Mol. Gen. Genet. 240:65-67, 1993; Long et al., PNAS 90:10370-10374, 1993)

La présente invention concerne donc également un polynucléotide tel que décrit ci-dessus comprenant un transposon *Impala* dans lequel est inséré un gène marqueur entre les deux ITRs de l'élément transposable sans affecter la fonctionnalité de la transposase, permettant ainsi de disposer d'un élément autonome et marqué. Tous les gènes marqueurs dont l'utilisation est envisagée pour repérer l'excision du transposon *Impala* peuvent également être utilisés, dans un mode préférentiel de l'invention, pour marquer ledit transposon. De préférence, le gène marqueur est inséré en aval de la séquence codant la transposase et en amont de l'ITR gauche (au site Nhel). L'insertion d'un gène marqueur dans le transposon *Impala* permet une meilleure sélection des mutants d'insertion. Alternativement, un gène marqueur tronqué peut être inséré dans le transposon *Impala*. L'utilisation d'un gène marqueur dépourvu de promoteur permet d'utiliser les polynucléotides de la présente invention comme piège à promoteur. L'utilisation d'un gène marqueur comportant un promoteur tronqué permet d'utiliser les polynucléotides de la présente invention comme piège de séquence activatrice.

La présente invention concerne enfin un polynucléotide tel que décrit ci-dessus comprenant un transposon *Impala* défectif c'est à dire un transposon dans lequel la transposase de l'élément *Impala* a été inactivée, en particulier par mutation, par délétion, par insertion d'un gène marqueur ou par remplacement par un gène marqueur. La transposition de cet élément *Impala* défectif est plus facilement contrôlée dans les méthodes de mutagenèse insertionnelle de la présente invention. La construction d'un élément *Impala* défectif dont la transposase est inactivée met en oeuvre des techniques classiques de biologie moléculaire connues de l'homme du métier (Sambrook et al., 1989). Dans un mode de réalisation de l'invention, le cadre ouvert de lecture codant pour la transposase de l'élément *Impala* est remplacé par un gène marqueur exprimé sous le contrôle d'un promoteur fonctionnel dans *Magnaporthe grisea*. La séquence codante de la transposase peut par exemple être remplacé par le gène de résistance à l'hygromycine, le gène de résistance au bialaphos ou le gène de la GFP exprimé sous le contrôle d'un promoteur hétérologue fonctionnel dans les champignons. Le transposon *Impala* défectif conserve ces séquences d'insertion (ITRs) et sa transposition peut donc être activé en trans par une transposase placé par exemple sur un plasmide replicatif.

Les polynucléotides de la présente invention sont de préférence insérés dans un vecteur. Ce vecteur peut être utilisé pour la transformation d'un organisme hôte tel qu'une bacterie par exemple et pour la réplication des polynucléotides de la présente invention dans cet organisme hôte. De préférence, les polynucléotides de la présente invention sont insérés dans un vecteur pour la transformation des champignons. Ces vecteurs sont utilisés pour la réplication ou pour l'intégration de ces polynucléotides dans le génome des champignons. Les vecteurs permettant la réplication et l'intégration de polynucléotides dans un organisme hôte sont bien connues de l'homme du métier.

### Mutagenèse insertionnelle et étiquetage génétique

La présente invention concerne également l'utilisation des polynucléotides décrits ci-dessus pour la préparation de mutants d'insertion de champignons et pour l'étude du génome de ces champignons.

La présente invention a donc également pour objet une méthode pour la préparation de mutants d'insertion de champignons comprenant les étapes suivantes:
- on transforme ledit champignon avec un polynucléotide selon l'invention comprenant un gène marqueur inactivé par l'insertion d'un transposon *Impala* dans des conditions permettant l'excision du transposon *Impala* dudit gène marqueur et sa réinsertion dans le génome du champignon ;
- on identifie les mutants d'insertion exprimant le gène marqueur.

Il est entendu que dans les méthodes selon l'invention le transposon *Impala* peut être modifié et notamment par l'insertion d'un gène marqueur ou de séquences d'activation. Dans un mode de réalisation préféré, le transposon *Impala* comprend un gène marqueur et on sélectionne les mutants d'insertion exprimant les deux gènes marqueurs.

Tout champignon peut être transformé avec un polynucléotide selon l'invention afin de préparer des mutants d'insertion de ce champignon. On citera notamment les ascomycètes, les basidiomycètes et les oomycètes. De préférence, l'invention concerne les champignons des genres *Alternaria, Aspergillus, Botrytis, Cladosporium, Claviceps, Colletotrichum, Erysiphe, Fusarium, Mycosphaerella, Phytophthora, Pseudocercosporella, Pyrenophora, Rhynchosporium, Sclerolinia, Stagonospora, Venturia* et *Ustilago*. On citera également les champignons des genres *Gaeumannomyces, Helminthosporium, Puccinia* et *Rhizoctonia*. Préférentiellement, l'invention concerne les champignons des genres *Magnaporthe* et *Penicillium*. Plus préférentiellement, l'invention concerne les champignons des espèces *Aspergillus fumigatus, Aspergillus nidulans, Botrytis cinerea, Erysiphe graminis, Mycosphaerella graminicola, Penicillium funiculosum* et *Stagonospora nodorum.* Encore plus préférentiellement, l'invention concerne *Magnaporthe grisea*.

Les techniques de transformation des champignons sont bien connues de l'homme du métier. On citera notamment la transformation de protoplastes utilisant le PEG, l'électroporation, la transformation par *Agrobacterium* (De Groot et al., Nature Biotechnology 16:839-842, 1998) ou les méthodes de bombardement par canon à particules (Chaure et al., Nature Biotechnology 18:205-207, 2000).

Les transformants sont ensuite criblés pour l'expression du gène marqueur afin d'identifier ou de sélectionner les mutants d'insertion résultant de la transposition de l'élément *Impala*. Le gène marqueur des polynucléotides de la présente invention permet l'identification ou la sélection de mutants d'insertion au moyen d'un crible phénotypique. A titre d'exemple, ce crible peut être une résistance à un antibiotique, une résistance à un composé chimique ou la mesure du niveau d'expression d'un gène rapporteur. Différents gènes marqueurs sont décrits plus en détail ci-dessus. Lorsque le gène *niaD* est utilisé comme gène marqueur dans un champignon nia- les mutants d'insertion sont sélectionnés grâce à leur aspect dense et aérien sur milieu minimum contenant du NaNO₃ comme seule source d'azote.

Afin d'analyser les mutants d'insertion ainsi obtenus il peut être intéressant de stabiliser le transposon afin d'éviter toute nouvelle transposition. Ce contrôle d'une nouvelle réinsertion du transposon peut être négligé si les mutants sont testés à un effectif proche ou en deçà du taux de transposition de l'élément de transposition. Afin de contrôler l'excision du transposon, un système à double composante peut être préparé (Hua-Van, 1998 ; Kempken and Kuck, 1998). Celui-ci implique la construction d'un élément *Impala* défectif dans lequel la transposase est inactivée, en particulier par mutation, par délétion ou par remplacement par un gène marqueur. Dans ce cas le transposon *Impala* défectif est mobilisé par une transposase dont l'expression est étroitement contrôlée permettant ainsi de stabiliser l'élément *Impala*.

La présente invention a donc également pour objet une méthode pour la préparation de mutants d'insertion de champignons caractérisée en ce qu'elle comprend les étapes suivantes:
- on transforme ledit champignon avec un polynucléotide comprenant un gène marqueur inactivé par l'insertion d'un transposon *Impala* défectif selon l'invention;
- on mobilise le transposon *Impala* défectif par une transposase dont l'expression est contrôlée dans des conditions permettant l'excision du transposon *Impala* défectif, sa réinsertion et sa stabilisation dans le génome du champignon;
- on identifie les mutants d'insertion exprimant le gène marqueur.

Les méthodes permettant de contrôler l'expression d'un gène tel que le gène de la transposase de l'élément *Impala* dans les champignons sont bien connues de l'homme du métier. Dans un mode de réalisation particulier, le champignon est transformé avec deux polynucléotides, le premier polynucléotide comprend le transposon *Impala* défectif alors que le deuxième polynucléotide comprend la séquence codante de la transposase de l'élément *Impala* sous le contrôle de son propre promoteur ou d'un promoteur hétérologue. La séquence codante de la transposase peut être placée sur un plasmide réplicatif ou sur un plasmide intégratif. Afin de contrôler l'expression de la transposase celle-ci peut être placée sous le contrôle d'un promoteur inductible. L'induction de l'expression de la transposase permet la transposition de l'élément *Impala* défectif et la préparation de mutants d'insertion puis le transposon est stabilisé lorsque la transposase n'est plus exprimée. Tout promoteur inductible fonctionnel dans les champignons peut être utilisé dans les méthodes de la présente invention. On pourra notamment utiliser le promoteur du gène de la nitrate réductase de *Magnaporthe grisea*, en effet ce promoteur permet l'expression du gène *nia* sur un milieu minimum en présence de nitrate comme seule source d'azote alors que l'expression de ce gène est totalement réprimée en présence d'ammonium (Lau et Hammer, 1996). Alternativement, la transposase est par exemple placée sur un plasmide réplicatif portant un marqueur de sélection, ce plasmide n'étant pas maintenu en absence de pression de sélection. Dans ce cas la transposase peut être exprimée sous le contrôle d'un promoteur constitutif ou de son propre promoteur. En présence d'une pression de sélection,le maintien du plasmide réplicatif permet l'expression de la transposase qui permet à son tour la transposition de l'élément *Impala* et l'obtention de mutants d'insertion. En absence de pression de sélection permettant le maintien du plasmide réplicatif, la transposase est perdue et le transposon est stabilisé dans les mutants. Les moyens nécessaires à la préparation d'un tel plasmide sont bien connus de l'homme du métier. A titre d'exemple, la transposase peut être placée dans le vecteur réplicatif pFAC1 contenant des extrémités télomériques de *Podospora* (Barreau et al., 1998).

La mutagenèse insertionnelle est un outil très performant pour l'identification de nouveaux gènes d'intérêt et pour l'étude de leur fonction. Dans un mode de réalisation préféré, une collection de mutants d'insertion est criblée pour un phénotype d'intérêt. Tout phénotype détectable peut être recherché dans les mutants d'insertion de la présente invention. On citera notamment des phénotypes concernant la biologie, la physiologie, le développement et la biochimie du champignon. De préférence, on prépare des mutants d'insertion de champignons pathogènes et les phénotypes recherchés dans ces mutants concernent la pathogenicité de ces champignons. Le crible phénotypique peut être basé sur une observation directe du champignon, sur une mesure d'activité enzymatique, sur la mesure d'une sensibilité à un fongicide ou encore sur l'étude de la virulence du champignon. Lorsqu'un mutant d'insertion possédant un phénotype d'intérêt a été identifié, le gène dans lequel ou à proximité duquel le transposon *Impala* s'est inséré est isolé. Le gène d'intérêt ainsi étiqueté par l'insertion de l'élément *Impala* est isolé à l'aide de techniques de biologie moléculaire bien connues de l'homme du métier. Parmi les techniques utilisées, on citera notamment les techniques d'amplification permettant l'amplification d'un polynucléotide lorsque seule la séquence d'une extrémité du polynucléotide est connue (dans ce cas la séquence du transposon intégré dans le génome). Ces techniques comprennent notamment la "PCR inverse" (Ochman et al., Genetics, 120:621-623, 1988; Williams, Biotechniques 7: 762-769, 1989), la "vectorette PCR" (Arnold et Hodgson, PCR Methods Appl. 1:39-42, 1991) et la "panhandle PCR" (Jones et Winistorfer, PCR Methods Appl. 2:197-203, 1993). Ces techniques permettent d'amplifier, de cloner et de séquencer les séquences flanquant le transposon *Impala* dans le génome du champignon. Ces séquences flanquantes sont ensuite utilisées pour isoler la totalité du gène inactivé par l'insertion du transposon.

La présente invention concerne donc également une méthode pour l'identification d'un gène associé à un phénotype détectable chez les champignons caractérisée en ce qu'elle comprend les étapes suivantes:
- on prépare des mutants d'insertion par insertion d'un transposon *Impala* dans le génome desdits champignons selon l'une des méthodes décrites ci-dessus;
- on sélectionne au moins un mutant d'insertion possédant ce phénotype détectable;
- on isole le gène dans lequel ou à proximité duquel s'est inséré le transposon *Impala*.

### Organismes hôtes

La présente invention concerne également un organisme hôte transformé avec un polynucléotide de la présente invention. Par organisme hôte, on entend en particulier selon l'invention tout organisme mono ou pluricellulaire, inférieur ou supérieur, en particulier choisi parmi les bactéries et les champignons. De manière avantageuse, les bactéries sont choisies parmi *Escherichia coli*. Dans un mode de réalisation préféré, l'invention concerne un champignon transformé avec un polynucléotide de la présente invention. De préférence, le champignon est choisi parmi les ascomycètes, les basidiomycètes et les oomycètes. Préférentiellement les champignons sont choisis parmi les champignons des genres *Alternaria, Aspergillus, Botrytis, Cladosporium, Claviceps, Colletoirichum, Erysiphe, Fusarium, Mycosphaerella, Phytophthora, Pseudocercosporella, Pyrenophora, Rhynchosporium, Sclerotinia, Stagonospora, Venturia* et *Ustilago*. On citera également les champignons des genres *Gaeumannomyces, Helminthosporium, Puccinia* et *Rhizoctonia*. Préférentiellement, les champignons sont choisi parmi *Magnaporthe* et *Penicillium*. De manière avantageuse les champignons sont choisis parmi les espèces *Aspergillus fumigatus, Aspergillus nidulans, Botrytis cinerea, Erysiphe graminis, Mycosphaerella graminicola, Penicillium funiculosum* et *Stagonospora nodorum.* De manière particulièrement avantageuse, l'organisme hôte est *Magnaporthe grisea*.

Le polynucléotide peut être intégré dans le génome du champignon ou placé sur un plasmide réplicatif. La présente invention concerne donc également un champignon dans le génome du quel est intégré un polynucléotide selon l'invention. La présente invention concerne également des mutants d'insertion de champignons filamenteux choisis parmi les champignons les genres *Magnaporthe* ou *Penicillium,* dans le génome du quel est intégré le transposon *Impala*.

La réinsertion d'*Impala* dans le génome du champignon permet de générer une collection de mutants d'insertion de ce champignon. Les mutants ainsi obtenus peuvent être employés pour l'étude du génome des champignons filamenteux.

Les exemples ci-après permettent d'illustrer la présente invention sans toutefois chercher à en limiter la portée. Toutes les méthodes ou opérations décrites ci-dessous dans ces exemples sont données à titre d'exemples et correspondent à un choix, effectué parmi les différentes méthodes disponibles pour parvenir au même résultat. Ce choix n'a aucune incidence sur la qualité du résultat et par conséquent, toute méthode adaptée peut être utilisée par l'homme de l'art pour parvenir au même résultat. La plupart des méthodes d'ingénierie des fragments d'ADN sont décrites dans "Current Protocols in Molecular Biology" Volumes 1 et 2, Ausubel F.M. et al. ou dans Sambrook et al., 1989.

### Description des figures

**Figure 1 :** Cartographie du plasmide pNi160
**Figure 2 :** Cartographie du plasmide pNiL160 (A) et du plasmide pAN301 (B) ayant servi à sa construction
**Figure 3 :** Analyse moléculaire des cotransformants obtenus en REMI BamHI à l'aide des vecteurs pNi160 et pCB1179. L'ADN des cotransformants est extrait, digéré par EcoRI et chargé sur gel d'agarose 1% dans le tampon TAE 1X (5µg par piste). Après migration et transfert sur membrane de nylon positive, les fragments d'ADN sont révélés par hybridation Southern à une sonde radioactive correspondant au fragment EcoRI de 2,7kb du gène *niaD* présent dans pAN301.
**Figure 4** : Analyse moléculaire des révertants nia+ C14-1 et C 14-2. L'ADN des révertants est extrait, digéré par EcoRI et chargé sur gel d'agarose 1 % dans le tampon TAE 1X (5µg par piste). Après migration et transfert sur membrane de nylon positive, les fragments d'ADN sont révélés par hybridation Southern.
   **A :** Analyse du révertant C14-1 à l'aide d'une sonde radioactive correspondant au fragment EcoRI de 2,7kb du gène *niaD* présent dans pAN301 (piste 1) ou à l'ORF codant la transposase d'*Impala* (piste 2).
   **B :** Analyse des révertants C14-1 et C14-2 après les avoir purifié en isolant des monospores nia+. Les profils des révertants C14-1 (pistes 3 et 4) et des révertants C14-2 (pistes 5 et 6) sont comparés au profil du cotransformant C14 nia- d'origine (pistes 1 et 2). La sonde utilisée correspond à l'ORF codant la transposase d'*Impala*.

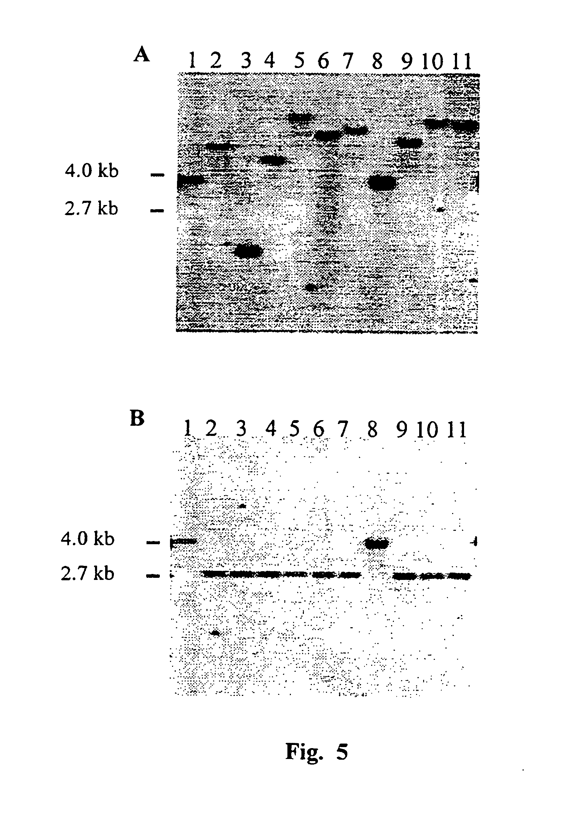

**Figure 5 :** Analyse moléculaire de révertants nia+ issus de deux cotransformants portant le vecteur pNiL160.L'ADN des cotransformants est extrait, digéré par EcoRI et chargé sur gel d'agarose 1% dans le tampon TAE 1X (5µg par piste). Après migration et transfert sur membrane de nylon positive, les fragments d'ADN sont révélés **A** : par hybridation Southern à une sonde radioactive correspondant à l'ORF codant la transposase d'*Impala*; **B:** par hybridation Southern à une sonde radioactive correspondant à un fragment EcoRI de 2,7kb du gène *niaD* présent dans pAN301. Piste 1 : ADN du cotransformant 8; pistes 2 à 7 : ADN des révertants du cotransformant 8; piste 8: ADN du cotransformant 6; pistes 9 à 11 : ADN des révertants du cotransformant 6.

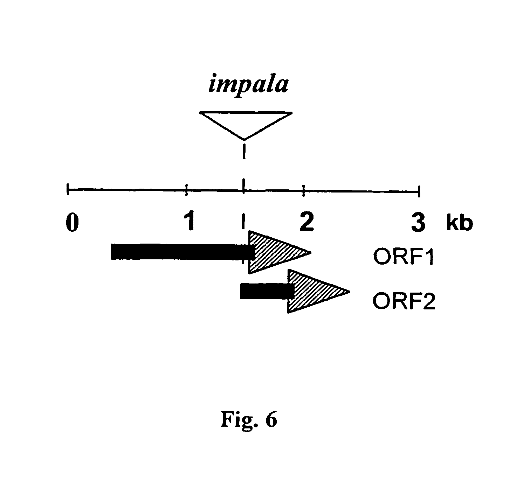

**Figure 6 :**Schématisation des ORFs interrompues par l'insertion *d'impala* chez le révertant non pathogène Rev77

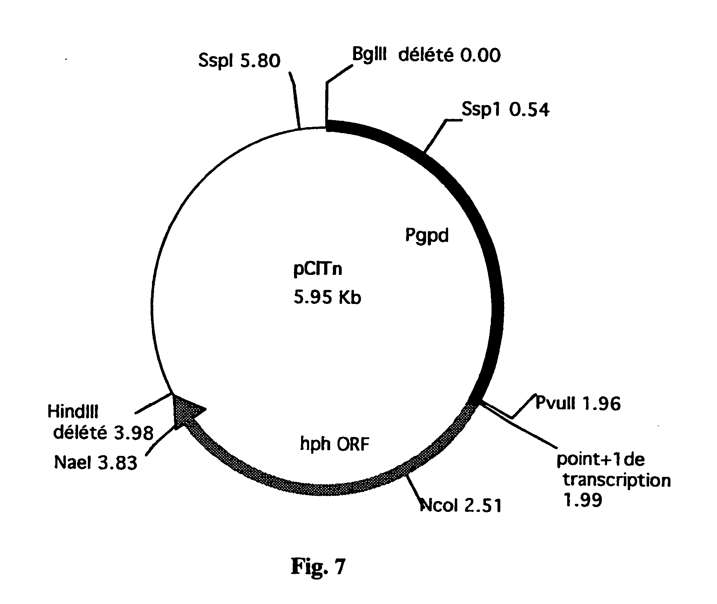

**Figure 7 :** Cartographie du plasmide pClTn

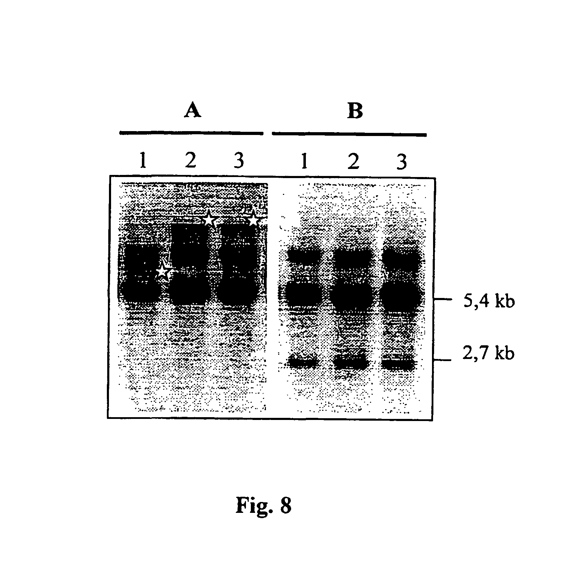

**Figure 8 :** Analyse moléculaire de révertants nia+ issus d'un cotransformant portant la construction pNiHYG. L'ADN de trois révertants indépendants (pistes 1 à 3) est extrait, digéré par EcoRI et chargé sur gel d'agarose 1% dans le tampon TAE 1X (5µg par piste). Après migration et transfert sur membrane de nylon positive, les fragments d'ADN sont révélés **A :** par hybridation Southern à une sonde radioactive correspondant à l'ORF codant la transposase *d'Impala;* B: par hybridation Southern à une sonde radioactive correspondant à un fragment EcoRI de 2,7kb du gène *niaD* présent dans pAN301. L'emplacement des étoiles signale la réinsertion de l'élément transpsosable.

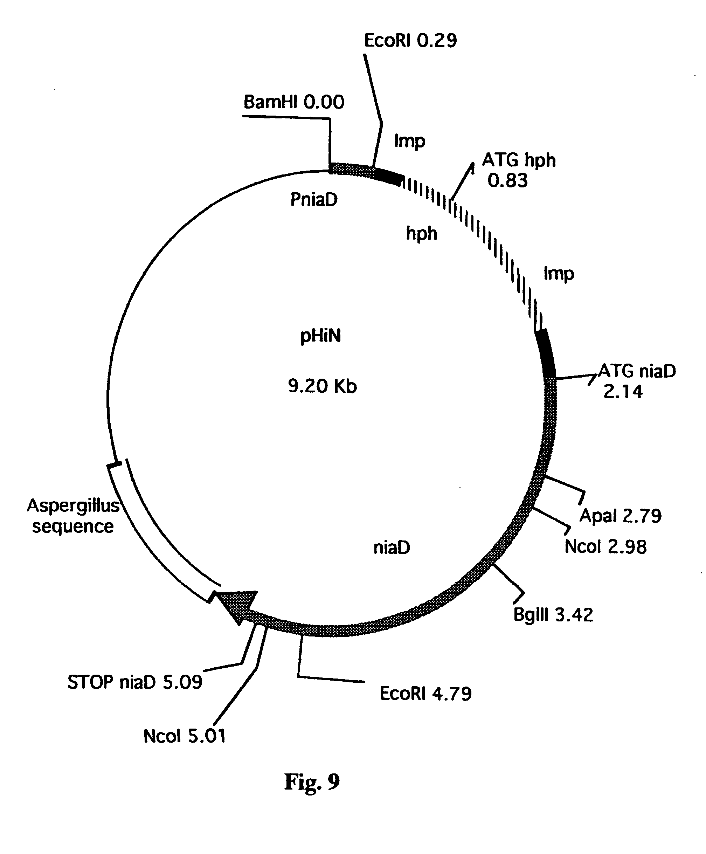

**Figure 9 :** Cartographie du plasmide pHIN

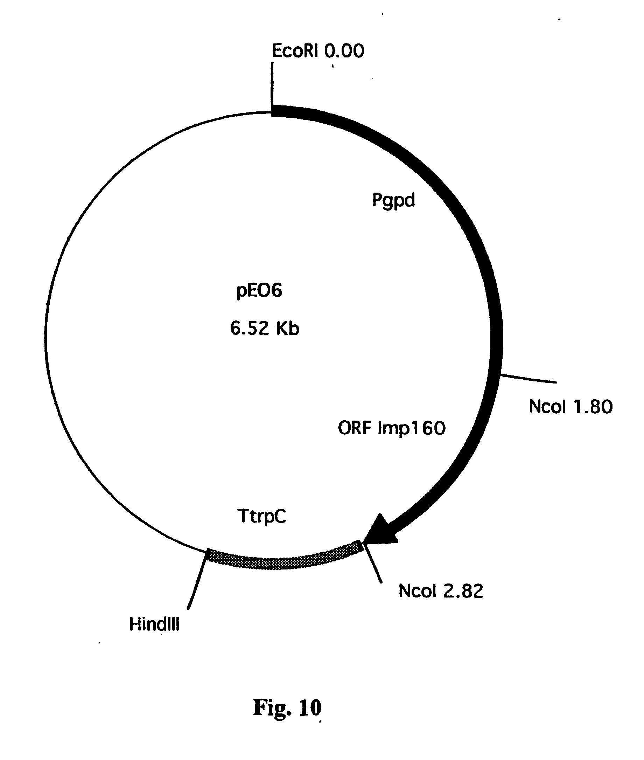

**Figure 10 :** Cartographie du plasmide pEO6

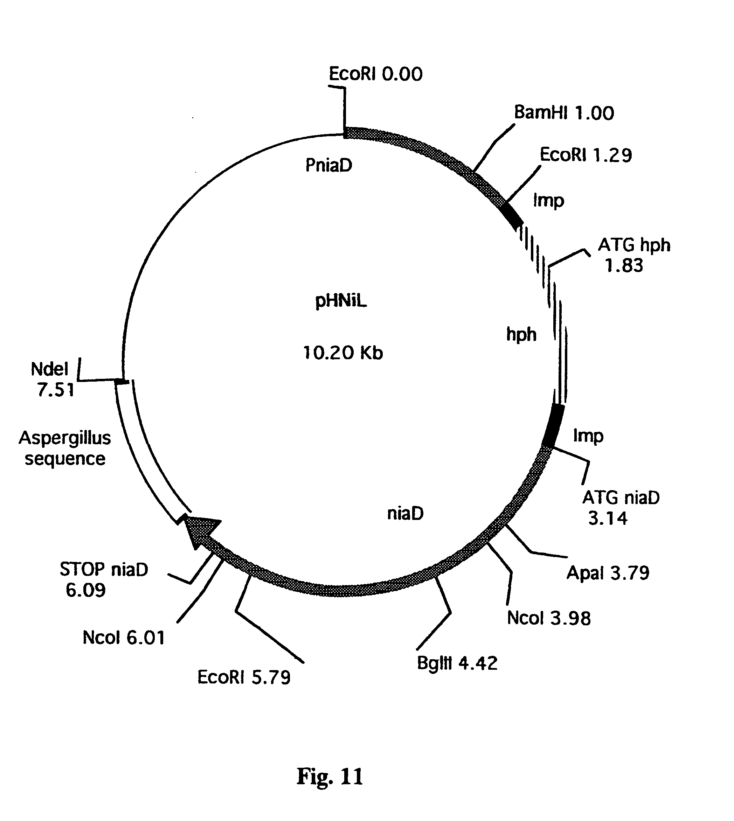

**Figure 11 :** Cartographie du plasmide pHNiL

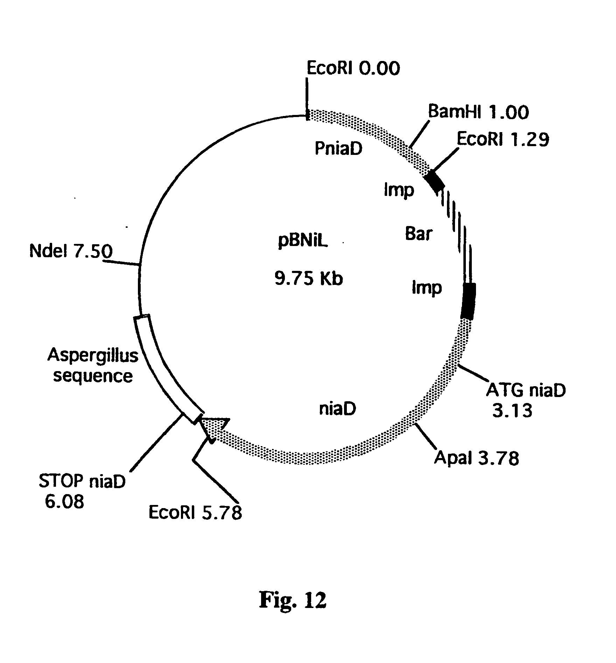

**Figure 12 :** Cartographie du plasmide pBNiL

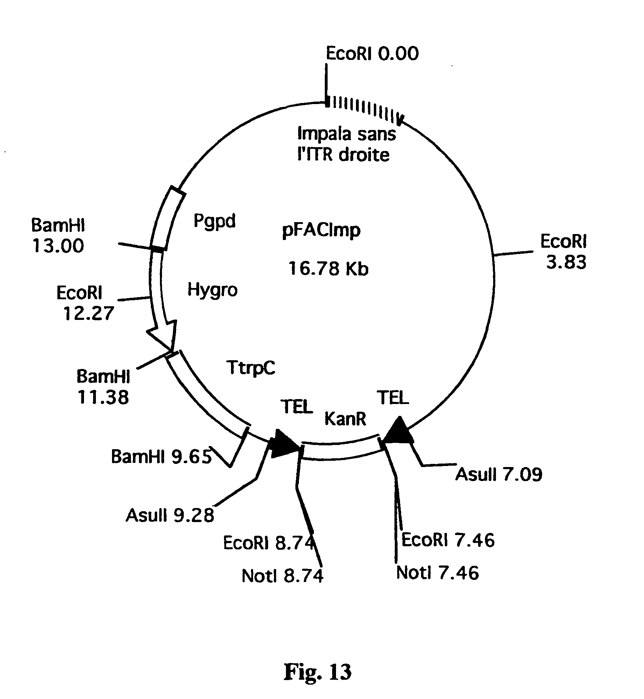

**Figure 13 :** Cartographie du plasmide pFACImp

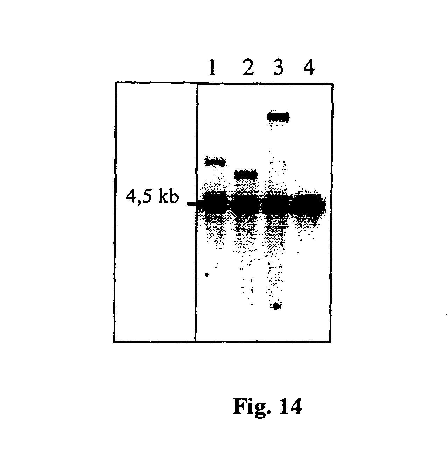

**Figure 14 :** Analyse moléculaire de révertants nia+ issus du cotransformant D1 obtenu après transformation par le système à double composante. L'ADN des révertants (pistes 1 à 4) est extrait, digéré par EcoRI et chargé sur gel d'agarose 1% dans le tampon TAE 1X (5µg par piste). Après migration et transfert sur membrane de nylon positive, les fragments d'ADN sont révélés par hybridation Southern à une sonde radioactive correspondant à un fragment amplifié à partir du gène *hph.*

### Exemples

### Exemple I

### Mutagénèse insertionnelle par une copie autonome d'Impala

### 1. Constructions disponibles.

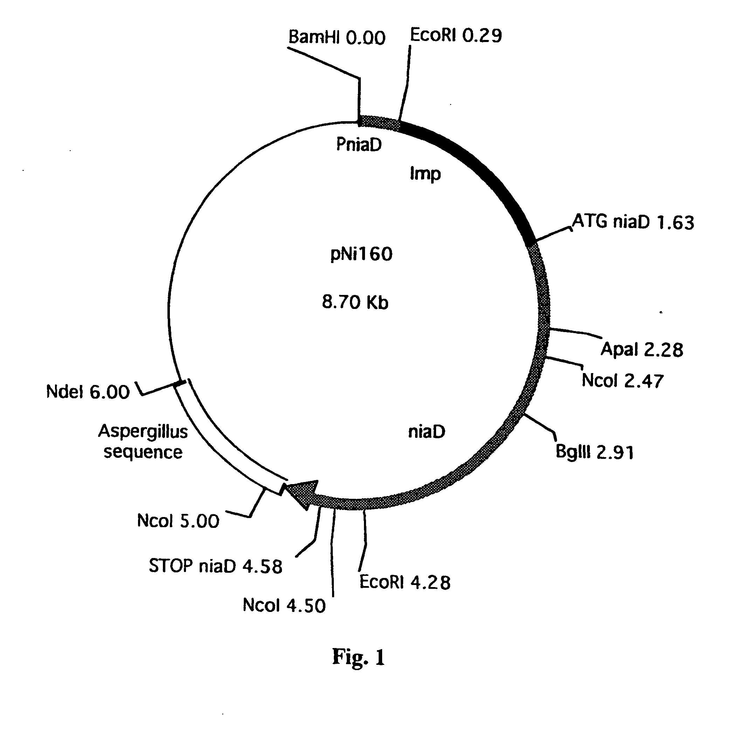

Le plasmide pNi160 possède la copie *Impala 160* intégrée dans la région promotrice du gène *niaD d'Aspergillus nidulans*, à 10 pb du codon ATG. Sa construction dérive du piège à transposon réalisé chez la souche F24 de *Fusarium oxysporum* transformée par le plasmide pAN301 (Malardier *et al*., 1989) possédant le gène de la nitrate réductase *d'Aspergillus nidulans* (Daboussi *et al*., 1992). La sélection de mutations spontanées dans le gène *niaD* a permis de caractériser chez le transformant TR7, portant une seule copie de pAN301, une insertion de 1,3 kb. Cette insertion est présente dans la région EcoRI-EcoRI de 2,7 kb de pAN301, ce qui a pour effet de générer un fragment EcoRI de 4 kb. Ce fragment a été cloné au site EcoRI de pUC19 après construction d'une banque génomique partielle et criblage par le fragment EcoRI de 2,7 kb provenant de pAN301 (Langin *et al*., 1995). Parallèlement le plasmide p11ΔNdeI a été construit à partir de pAN301 en délétant un fragment NdeI de 7,8 kb placé en aval du gène *niaD*, ainsi qu'un fragment EcoRI-BamHI de 1 kb correspondant à la plus grande partie du promoteur du gène *niaD* (Langin *et al*., 1990). Le remplacement du fragment EcoRI de 2,7 kb présent dans p11ΔNdeI par le fragment de 4kb comportant le fragment de 2,7 kb de la nitrate reductase et l'élément *Impala 160* a permis d'obtenir le plasmide pNi160 dans lequel l'élément est inséré dans la région promotrice du gène *niaD*, présentant ici une taille de 0,3 kb (figure 1)

### 2. Constructions réalisées.

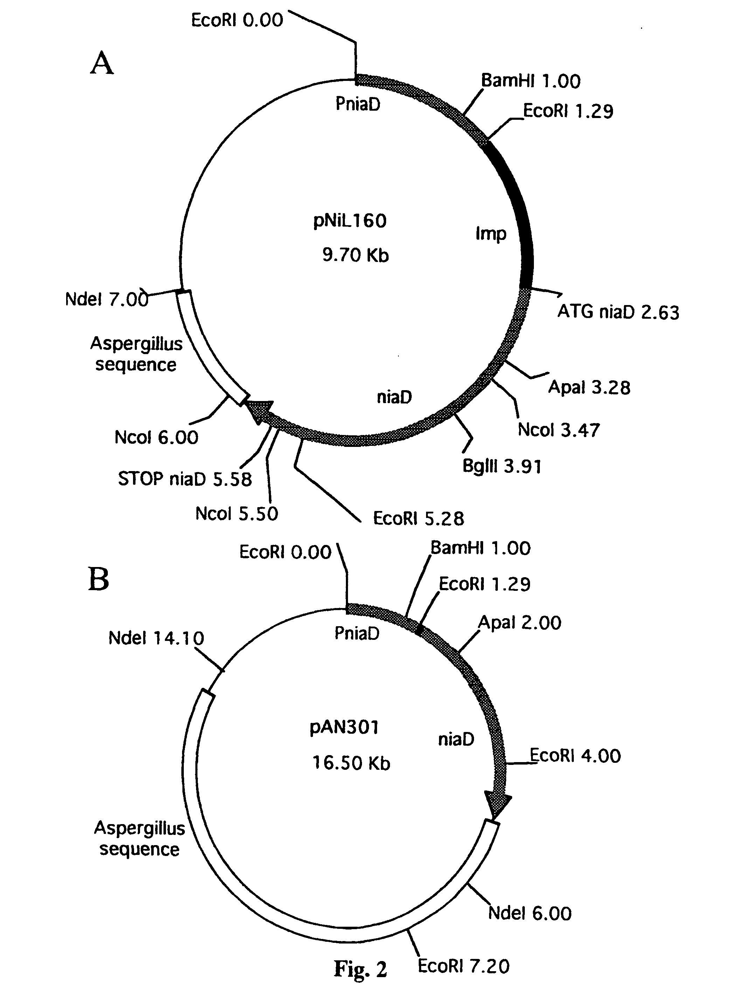

Le plasmide pNiL160 dérive du plasmide pNi 160, par l'addition d'un fragment de 1 kb du promoteur du gène *niaD* présent dans pAN301. Pour ce faire, le plasmide pAN301 possédant 1,3 kb de promoteur *niaD* a été délété du fragment NdeI de 7,8 kb présent en aval de ce gène donnant le plasmide intermédiaire pAN301ΔNdeI. Puis son fragment BamHI-ApaI de 1 kb a été remplacé par un fragment BamHI-ApaI de 2,3 kb, provenant du plasmide pNi160 et possédant la même portion du gène *niaD* que le fragment de 1 kb plus l'élément *Impala 160* (figure 2).

### 3. Transformation de Magnaporthe grisea

La souche de *Magnaporthe grisea* G 11.174 présente une mutation ponctuelle dans le gène de la nitrate reductase responsable de son phénotype nia-. Son obtention est décrite dans l'article Daboussi *et al*., 1989. Elle est repiquée sur un milieu solide à base de farine de riz, à partir duquel il est possible de récolter des conidies du champignon. Le milieu liquide TNKYE préparé d'après le milieu B de Tanaka (Ou *et al*., 1985) permet de récolter du mycélium en vue d'extraire l'ADN génomique ou d'obtenir des protoplastes selon les protocoles décrits par Sweigard *et al*. (1990) et Sweigard *et al*. (1992). Le milieu gélosé TNK (agarose ultra pure, 8 g.l⁻¹) dépourvu d'extrait de levure (milieu MNO₃) permet de différencier la souche nia- G 11.174 de la souche nia+ G 11.25 ; la première présente un phénotype ras et filamenteux tandis que la seconde est dense et aérierme.

### 3.1. Transformation avec le plasmide pNi160

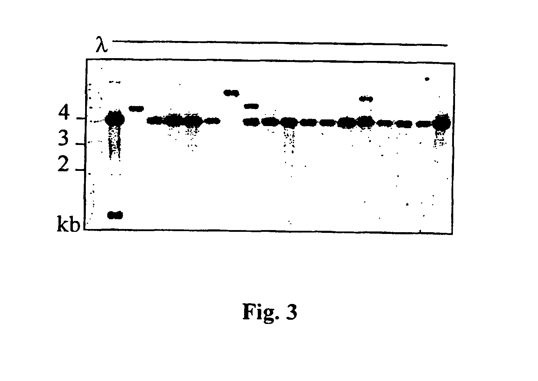

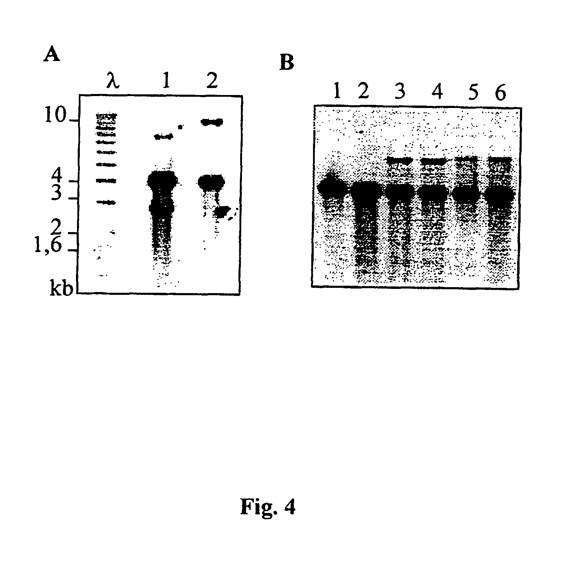

Des protoplastes de la souche G11.174 ont été cotransformés par le plasmide pNi 160 (apportant *Impala 160*) et le plasmide pCB 1179 (Sweigard *et al.,* 1997) conférant la résistance à l'hygromycine. La méthode de transformation est décrite par Sweigard *et al*., 1992 et a été réalisée en présence de 4 unités d'enzyme BamHI (REMI : restriction enzyme mediated integration ; Sweigard *et al*., 1998) et 1 µg de chaque plasmide. Les protoplastes sont sélectionnés sur un milieu TNKYE où le glucose a été remplacé par du saccharose (400 µg.l⁻¹) et additionné d'hygromycine à raison de 240 µg.ml⁻¹. Afin de sélectionner les cotransformants, les colonies résistantes à cet antibiotique ont été analysées, après extraction de leur ADN génomiques, par amplification à l'aide des amorces SPE5 (5'AGAACACAACCCTGCCACGG 3') et SPE3 (S'TCCGGGCCGTATGCACAGAG 3') spécifiques du transposon *Impala* et générant un produit d'amplification de 573 pb. L'ADN des cotransformants a été digéré par EcoRI et analysé en Southern-blot (figure 3) en utilisant comme sonde un fragment EcoRI de 2,7 kb du gène niaD (sonde 2,7 kb) présent dans pAN301 (Malardier *et al*., 1989). Cette étude a permis de sélectionner 35 cotransformants possédant au moins une bande de 4 kb représentant la quasi totalité du gène *nia d'Aspergillus nidulans* introduit via pNi 160. Ces cotransformants ont été cultivés sur milieu solide à base de farine de riz, pendant 10 à 14 jours, et les spores récoltées dans de l'eau. Après comptage, elles ont été ensemencées sur milieu gélosé MNO₃ à raison de 10⁵-10⁶ spores par boîtes. Des expériences reconstituant cette étape de sélection des révertants nia+ ont été réalisées. Nous avons ainsi observé que le milieu MNO₃ permet de détecter des colonies nia+ lorsque 10 spores sauvages (G11.25) sont mélangées à 10⁶ spores du mutant nia- G11.174 et incubées 14 jours à 26°C. Après 1 mois de culture à 26°C seulement un cotransformant (cotransformant C14) a permis d'obtenir deux colonies (C14-1 et C14-2) présentant un phénotype aérien. Ces colonies révertantes ont été récupérées et analysées par PCR à l'aide des amorces C1 (5'CGCTGCGAATTCTTCAGT 3') et niaX (5'CTAGACTTAGAACCTCGG 3') encadrant le site d'insertion d'*Impala160* dans le promoteur du gène *niaD*. L'amplification d'un produit de 200 pb révèle la présence de noyaux dans lesquels l'excision du transposon s'est produite. Afin d'obtenir des colonies homogènes, des conidies des révertants C14-1 et C14-2 ont été isolées sous la loupe binoculaire et cultivées séparément. Leur analyse par Southern-blot, à l'aide d'une sonde correspondant à l'ORF d'*Impala* permet de mettre en évidence la réinsertion de l'élément chez les deux révertants (figure 4). La cicatrice laissée par l'excision du transposon a été séquencée après clonage du produit PCR de 200 pb dans le vecteur pGEM-T easy (Promega). La cicatrice du révertant C14-1 est *CTG*TA et celle de C14-2, *CAG*TA. Ces cicatrices sont identiques à celles le plus souvent laissées par *Impala* lors de son excision chez *Fusarium oxysporum* (Langin *et al*., 1995). En culture sur milieu gélosé MNO₃, ces révertants présentent un phénotype intermédiaire entre celui des souches G 11.174 et G 11.25 suggérant que le gène *niaD* présent dans la construction pNi 160 ne permet pas de complémenter de façon optimale la mutation de la souche G 11.174. Pour tester cette hypothèse, des protoplastes de cette souche ont été transformés par les vecteurs pAN301 (3µg) ou pAN301ΔNdeI (3 µg) possédant le gène *niaD* sous contrôle de 1,3 kb de promoteur et en présence de pCB 1179 (3 µg). Après étalement des protoplastes et incubation à 30°C pendant 10 jours, sur milieu MNO₃ additionné d'hygromycine (240 µg.ml⁻¹) et où le glucose a été remplacé par du saccharose (400 µg.l⁻¹), des colonies à phénotype nia+ apparaissent. En revanche, aucune complémentation n'a été observée lorsque le vecteur p11ΔNdeI possédant le gène *niaD* sous contrôle d'un fragment de promoteur de 0,3 kb (comme dans le cas de pNi160) a été utilisé.

Ces expériences démontrent que le promoteur tronqué présent dans pNi160 est incapable de complémenter la mutation de la souche G 11.174. La sélection de deux révertants (C14-1 et C14-2) avec cette construction n'est pas due à l'activité intrinsèque du fragment de promoteur de 0,3 kb mais peut s'expliquer si l'on considère que chez le cotransformant C14, le gène *niaD* s'est inséré dans une région où il bénéficie de séquences activatrices. Ceci suggère que p11ΔNdeI pourrait être utilisé dans le but de détecter des régions activatrices dans le génome de *Magnaporthe grisea*.

### 3.2. Transformation avec pNiL160 (selon l'invention)

Des protoplastes de la souche G 11.174 ont été cotransformés par la nouvelle construction pNiL160 (1 µg), possédant le gène *niaD* sous contrôle d'un promoteur entier de 1,3 kb et pCB1179 (1 µg), en présence de 40 unités d'enzyme NdeI. Les cotransformants ont été criblés par amplification, à l'aide des amorces SPE5 et SPE3, puis ensemencés sur milieu riz en vue d'obtenir des conidies. Celles-ci ont été étalées sur milieu MNO₃ (de l'ordre de 10⁵-10⁶ spores par boîte) afin d'identifier des révertants. L'expérience conduite sur 19 cotransformants a permis d'obtenir des révertants dans 100% des cas ; certains apparaissent dès 10 jours de culture à 26°C. Le nombre de révertants oscille entre 2 et 83 selon le cotransformant considéré. 53 révertants appartenant à 8 cotransformants différents ont été analysés en Southern-blot grâce à une sonde correspondant à la totalité de la phase codante d'*Impala*. La figure 5 illustre la mobilié du transposon *impala* chez 9 révertants pris au hasard. Le pourcentage de réinsertions d'*Impala* chez *M. grisea* tiré de cette expérience atteint 74 %. Parmi ces réinsertions, 95 % d'entre elles sont différentes. Plus particulièrement, des cotransformants donnant 100% de réinsertion, dont toutes sont différentes, ont été identifiés.

Ces résultats démontrent que la construction pNiL160 permet, à la différence de pNi160, la sélection chez *Magnaporthe grisea* de nombreux révertants ayant une nouvelle insertion du transposon.

### Exemple II

### Préparation de mutants d'insertion chez Magnaporthe grisea

L'analyse en Southern-blot de 18 révertants nia+ obtenus à partir du cotransformant 6 (CTRF6) portant une seule copie du plasmide pNLi160 a montré que 100% d'entre eux présentent *Impala* réinséré dans le génome. Ce cotransformant a été choisi pour générer une collection de 350 révertants nia+. Le cotransformant est cultivé pendant 14 jours sur milieu riz. Les spores sont récoltées dans 3 ml d'eau distillée par boîte de Pétri, filtrées sur verre fritté afin d'éliminer les débris mycéliens et étalées sur milieu NaNO₃ à raison de 10⁶ spores par boîte (format 12x12 cm). Les boîtes sont mise à incuber à 26°C. Les révertants nia+ apparaissent 16 à 21 jours plus tard; ils sont repiqués sur milieu NaNO₃ cultivés 14 jours et les spores étalées sur milieu eau gélosée (2% agarose dans H20). Après 8 heures à 26°C, une spore de chaque révertant est individuellement repiquée sur milieu NaNO₃ afin de purifier le révertant et de vérifier son phénotype nia+.

### Exemple III

### Caractérisation de mutants d'insertion portant le transposon Impala

L'observation des 350 révertants générés à partir de CTRF 6 a permis de détecter un révertant (Rev2) présentant une croissance, sur milieu riz et milieu NaNO₃, moins importante que CTRF6, ainsi qu'une coloration brun foncé différente de la couleur grise de du cotransformant. Afin de caractériser les séquences flanquant l'insertion d'*Impala*, 3µg d'ADN génomique de Rev2 ont été digérés par HindIII et analysés par Inverse-PCR. Après digestion, l'ADN est soumis à une étape d'extraction au Phénol/Chloroforme puis précipité à l'acétate d'ammonium 7,5M. Le culot ainsi obtenu est repris dans 40µl d'eau MilliQ. Une ligation est réalisée sur 8 µl d'ADN digéré puis l'ADN est soumis comme précédemment à une extraction au Phénol/Cloroforme et précipité à l'acétate d'ammonium 7,5M. L'ADN est repris dans 10 µl et utilisé dans sa totalité pour une étape de PCR à l'aide des amorces ImpE5' (S'GGCATTGAAAACGCGGTCCC3') et ImpE3' (5'CAGCAGCAAAACAGCTGCCC3') choisies sur la séquence du transposon *Impala,* placées de façon divergente. Le séquençage du produit IPCR a permis de montrer que le transposon est inséré dans une phase de lecture ouverte au niveau d'un dinucléotide TA dupliqué. L'interrogation des banques de données à l'aide du programme tblastx (Altschul et al., 1990) a révélé une très forte homologie entre cette séquence mutée et une famille de protéines impliquée dans la réparation de l'ADN et plus particulièrement avec la protéine MLH 1 de *Saccharomyces cerevisiae* (Prolla et al., 1994).

Parallèlement, afin de rechercher des mutants de pathogénie, les 350 révertants de la collection ont été testés sur feuilles de riz et d'orge. Les révertants sont cultivés sur milieu riz pendant 14 jours. Les spores produites sont récoltées en grattant le mycélium en présence de 3 ml d'eau puis comptées sous cellule de Thomas. Les suspensions de spores sont ajustées à 10⁵spores/ml et badigeonnées à l'aide d'un coton de tige sur des morceaux de feuilles de riz (cv Sariceltik) et d'orge (cv Express) placées, en survies, sur milieu gélosé (1% agar) additionné de Kinetine (1 ml/l d'une solution stock à 2mg/ml dans l'éthanol).Après incubation pendant 4 jours à 26°C, les symptômes sont comparés à ceux occasionnés par la souche sauvage G 11.174. Le révertant 77 s'est avéré non pathogène. Les séquences flanquant l'insertion d'*Impala* chez ce mutant ont été récupérées par Inverse-PCR, dans les conditions décrites ci-dessus, après digestion de l'ADN génomique par BamHI. Le produit récupéré confirme à nouveau l'insertion du transposon au niveau d'un dinucléotide TA dupliqué placé dans une phase de lecture ouverte. L'interrogation des banques de données avec la séquence de ce produit n'a pas permis de mettre en évidence d'homologie significative. Ce produit a été utilisé pour sonder une banque cosmidique de *M. grisea*. Un cosmide hybridant avec le produit amplifié a été cloné, cartographié et séquencé en partie. Il apparaît ainsi que le transposon *Impala* est inséré dans deux ORFs potentielles (ORF1 et ORF2) suivant la phase de lecture considérée (Figure 6). Un fragment SalI/NotI de ce cosmide a été sous-cloné dans un vecteur pCB 1531 (vecteur pCB1531*) portant le gène Bar conférant la résistance au Bialaphos (Sweigard et al., 1997). Des protoplastes de Rev77 ont été obtenus et transformés par 3µg du plasmide pCB1531* ou par 3µg du plasmide pCB1531 comme témoin et déposés sur un milieu TNKYE saccharose (400g.l⁻¹) additionné de Bialaphos (30-40µg.ml⁻¹). Les colonies résistantes sont repiquées sur TNKYE glucose additionné de Bialaphos (30 µg.ml⁻¹) puis mise à sporuler pendant 14 jours sur milieu riz. Les spores sont récoltées, les suspensions calibrées à 10⁵ spores.ml⁻¹ puis testées quant à leur pathogénie comme il a été décrit ci-dessus. Comme attendu les transformants portant pCB1531 restent non pathogènes, à l'instar de Rev77, tandis que des colonies portant pCB1531* redeviennent pathogènes. Ces résultats démontrent que le transposon *Impala* a permis d'étiqueter un nouveau gène impliqué dans le pouvoir infectieux de *M. grisea*.

### Exemple IV

### Intégration du transposon Impala dans le promoteur du gène gpd

Un plasmide permettant le clonage d'*Impala* dans un promoteur contrôlant l'expression du gène de résistance à l'hygromycine *(hph)* a été construit. Une double digestion BglII-HindIII du vecteur pAN7.1 (Punt *et al*., 1987) permet de libérer un fragment de 3988 pb contenant la totalité de l'ORF codant la phase codante du gène *hph* ainsi que le promoteur du gène *gpd* délété de ses 137 premières paires de base et le terminateur du gène *TrpC*. Les extrémités cohésives de ce fragment ont été transformées en bouts francs grâce à l'action de l'ADN polymérase. Ce fragment a été par la suite ligué à un fragment PvuII de 2,5 kb, issu du plasmide pBluescript SK-, et portant l'origine de réplication ainsi que le gène de résistance à l'ampicilline. Le plasmide qui en résulte permet l'obtention chez *Magnaporthe grisea* de transformants résistants à l'hygromycine. Ce vecteur nommé pCITn (figure 7) possède un site PvuII unique situé à 30 pb en amont du point +1 de transcription dans lequel peut être cloné *Impala* ou tout autre transposon.

### Exemple V

### Mutagénèse insertionnelle par une copie autonome et marquée

Afin d'obtenir un élément autonome permettant de sélectionner, par un crible phénotypique, les révertants dans lesquels le transposon *Impala* s'est réinséré, le gène de résistance à l'hygromycine a été cloné entre les deux ITRs de l'élément, en aval du codon stop du cadre de lecture codant la transposase. Pour ce faire, la cassette de résistance à l'hygromycine a été récupérée à partir du plasmide pCB 1004 (Sweigard et al., 1997) par une digestion SalI et les extrémités rendues franches par traitement à la Klenow. Cette cassette est liée avec le plasmide pNi160, linéarisé au site NheI et traitée à la Klenow. Le plasmide résultant est digéré par les enzymes BamHI et ApaI. Le fragment de 2285 pb contenant le transposon *Impala* modifié est récupéré et lié avec le fragment de 7397 pb de pAN301ΔNdeI digéré par ces mêmes enzymes. Il en résulte un plasmide de 9682 pb portant dans le promoteur de *niaD*, de taille 1,3 kb dans lequel est inséré, 8 pb en amont du codon initiateur de la nitrate réductase, le transposon *Impala* marqué par la cassette de résistance à l'hygromycine inséré dans le sens de transcription de *niaD* (plasmide pNiHYG) ou dans le sens contraire (plasmide pNiGYH).

Des protoplastes de la souche G11.174 de *M. grisea* ont été transformés par 3 µg du plamide pNiHYG ou 3 µg du plasmide pNiGYH. La sélection de révertants nia+ a été réalisée sur ces transformants dans les conditions déjà décrites au préalable. L'analyse de trois révertants nia+ d'un même cotransformant par Southern-blot montre que le transposon *Impala* ainsi marqué reste autonome, c'est à dire qu'il est capable de s'exciser du gène *niaD* et de se réinsérer dans le génome (figure 8).

### Exemple VI

### Mutagénèse insertionnelle par une copie défective et mobilisable d'Impala.

L'exploitation d'un système de mutagénèse à deux composantes nécessite de montrer que l'élément transposable peut être activé en trans. Pour cela la transposase est placée dans un premier temps, sous le contrôle d'un promoteur constitutif. Dans un second temps, la stabilisation de l'élément défectif requiert l'utilisation d'un promoteur inductible contrôlant l'expression de la transposase ou d'un plasmide réplicatif portant la transposase sous le contrôle de son propre promoteur ou d'un promoteur constitutif. L'exploitation du promoteur du gène de *Magnaporthe grisea* codant la nitrate réductase en tant que promoteur inductible semble spécialement indiquée. Lau et Hamer (1996) ont montré par hybridation northem à l'aide d'une sonde correspondant à un clone possédant le gène de la nitrate réductase de *Magnaporthe grisea*, qu'il s'exprime en présence de nitrate comme seule source d'azote alors qu'il est totalement réprimé en présence de glutamine. Positionner la transposase d'*Impala* sous le contrôle du promoteur du gène *nia* de *Magnaporthe grisea* devrait permettre la synthèse de l'enzyme et par conséquent l'excision de l'élément défectif dans les conditions de sélection des révertants (milieu MNO₃) et l'inhibition de sa production, une fois le révertant obtenu, lors de sa culture sur milieu riche (présence d'ammonium ou de glutamine).

### 1. Constructions disponibles.

Le plasmide pHIN dérive de pNi160. Dans celui-ci, la transposase codée par l'élément *Impala* a été remplacée par le gène de résistance à l'hygromycine (gène *hph)* sous contrôle du gène *TrpC d'Aspergillus nidulans* (figure 9). Sa construction est décrite dans Hua-Van, 1998. La présence du gène *hph* au sein des ITRs du transposon permet de s'assurer de l'intégration de l'élément défectif dans le génome du révertant obtenu.

Le plasmide pEO6 dérive du plasmide pNOM102 après substitution de l'ORF codant la β-glucuronidase par l'ORF codant la transposase *d'Impala* obtenue par PCR à l'aide d'amorces contenant un site NcoI. Ce plasmide permet l'expression de la tranposase sous le contrôle du promoteur constitutif *gpd* et du terminateur *TrpC d'Aspergillus nidulans* (figure 10).

### 2. Constructions réalisées.

Le plasmide pHNiL dérive du plasmide pHIN. Il a été construit en remplaçant le fragment BamHI-ApaI de 1 kb de pAN301ΔNdeI par le fragment BamHI-ApaI de 2,8 kb provenant de pHIN et apportant la copie *Impala* défective et marquée par *hph.* Comme dans pNiL160, le gène de la nitrate réductase (*niaD*) est sous le contrôle de son promoteur de taille 1,3 kb (figure 11). D'après nos résultats, la construction de ce plasmide est nécessaire pour sélectionner l'excision de l'élément défectif, par restauration de l'activité nitrate réductase chez *Magnaporthe grisea*.

Le plasmide pBNiL possède également un élément défectif marqué ici par le gène *Bar*. Pour construire ce vecteur, le fragment BamHI/NcoI de pNiL 160 (2472 pb) contenant le transposon *Impala* bordé par des séquences du gène *niaD* est lié au fragment BamHI/AflIII de pUC 19 (2298 pb) portant l'origine de réplication du plasmide et le gène de résistance à l'ampicilline. On délète sur ce plasmide un fragment XhoI/StyI de 891 pb correspondant à une partie de la transposase d'*Impala*. Les extrémités du plasmide ainsi linéarisées sont rendues franches par action de la Klenow et liées avec le gène de résistance au Bialaphos (Promoteur Trpc::Bar, 940 pb) obtenu par digestion SalI du plasmide pCB1635 (Sweigard et al., 1997) et action de la Klenow. Le plasmide qui en résulte est digéré par BamHI et ApaI et lié avec le fragment BamHI/ApaI de 7397 pb du plasmide pAN301ΔNdeI. Il en résulte le plasmide pBNiL dans lequel le transposon est défectif, marqué par le gène Bar et inséré dans le promoteur (1,3 kb) du gène *niaD* (figure 12).

Le plasmide pFACImp porte le transposon *Impala* sans son ITR droite afin qu'il ne puisse plus transposer mais qu'il reste la source de la transposase. L'élément est excisé du plasmide pNi160 par une double digestion EcoRI/NheI, les extrémités sont rendues franches par l'action de la Klenow, puis le fragment est cloné au site BglII de pFAC1 (figure 13).

### 3. Utilisation de ces plasmides chez Magnaporthe grisea.

Des protoplastes de *Magnaporthe grisea* G11.174 ont été transformés par le plasmide pHNiL ou cotransformés par les plasmides pHNiL et pEO6. La méthode de transformation est décrite par Sweigard *et al*. (1992) et a été réalisé avec 1 µg de chaque plasmide. Les protoplastes sont sélectionnés sur un milieu TNKYE où le glucose a été remplacé par du saccharose (400 µg.l⁻¹) et additionné d'hygromycine à raison de 240 µg.ml⁻¹. Les transformants pHNiL sont directement sélectionnés grâce à la présence du marqueur de résistance dans l'élément défectif. Les cotransformants sont isolés parmi les colonies résistantes à l'hygromycine, après extraction de leur ADN génomique, par amplification à l'aide des amorces SPE5 décrites en IV.3. Cette étude menée sur 12 colonies résistantes à l'hygromycine a permis d'isoler 4 colonies portant également pE06. Après sporulation sur milieu solide à base de farine de riz, les spores (10⁵-10⁶) de ces cotransformants ainsi que de 6 transformants portant pHNiL ont été étalées sur milieu MNO₃ afin de sélectionner des révertants nia+ comme décrit en IV.3. Aucun de 6 transformants portant pHNiL n'ont donné de tels révertants. Ceci montre que la copie défective *d'Impala* ne peut pas être mobilisée par un transposon endogène à *Magnaporthe grisea*. Parmi les 4 cotransformants pHNiL/pEO6, deux d'entre eux donnent des colonies aériennes (cotransformants D1 et D9). L'analyse en Southern de 6 révertants issus du cotransformant D1, après digestion de leur ADN génomique par EcoRI et hybridation avec une sonde du gène *hph* de 868 pb obtenue à l'aide des amorces hyg1 (5'AGCCTGAACTCACCGCGACG 3') et hyg4 (5'CGACCCTGCGCCCAAGCTGC 3') permet de caractériser la réinsertion de l'élément défectif pour 4 d'entre eux (figure 14). Parmi ceux-ci deux révertants possèdent deux insertions de l'élément. Cette analyse permet de montrer que l'élément défectif peut être mobilisé, chez *Magnaporthe grisea*, par la transposase *d'Impala* apportée en trans.

### 4. Autres constructions.

Il s'agit, dans un premier temps, de construire un plasmide où la transposase *d'Impala* est sous le contrôle du promoteur du gène *nia* cloné chez *Magnaporthe grisea* (pNiaI). Ce plasmide est utilisé en combinaison avec pHINL ou tout autre plasmide dérivé de celui-ci où le promoteur *niaD d'Aspergillus nidulans*, inactivé par l'insertion de l'élément défectif, contrôle l'expression des gènes marqueurs utilisés pour la sélection des révertants. Pour faciliter la sélection des cotransformants un marqueur de résistance devra être ajouté à pNiaI, différent de celui présent dans le plasmide portant la copie défective de l'élément.

Dans un second temps, la transposase pourra être clonée sous le contrôle d'un promoteur constitutif, et plus précisément sous le contrôle du promoteur du gène *gpdA*, dans le vecteur pFAC1 portant un marqueur de sélection différent de celui présent dans l'élément défectif ou dans le plasmide pHNiL.

### Références

Altschul SF, Gish W, Miller W, Myers EW and Lipman DJ (1990) Basic local alignment search tool. J. Mol. Biol. 215,403-410
Barreau C, Iskandar M, Turcq B and Javerzat JP (1998) Use of linear plasmid containing telomeres as an efficient vector for direct cloning in the filamantous *fungus Podospora anserina*. Fungal Genetics and Biology 25, 22-30
Calvi BR, Hong TJ, Findley SD and Gelbart WM (1991) Evidence for a common evolutionary origin of inverted repeat transpsosons in *Drosophila* and *plants : hobo, Activator* and *Tam3.* Cell 66, 465-47
Capy P, Bazin C, Higuet T and Langin T (1998) Dynamics and evolution of transposable elements. Molecular Biology Intelligence unit. Springer-Verlad ed. Landes Biosciences. Heidelberg.
Doak TG, Doerder FP, Jahn CL ans Herrick G (1994) A proposed superfamilly of transposase genes : transposon-like elements in ciliated protozoa and a common »D35E » motif. Proc. Natl. Acad. Sci. USA 91, 942-946
Daboussi MJ, Djeballi A, Gerninger C, Blaiseau PL, Bouvier I, Cassan M, Lebrun MH, Parisot D and Brygoo Y (1989) Transformation of seven species of filamentous fungi using the nitrate reductase gene *of Aspergillus nidulans*. Curr. Genet 14, 453-456 Daboussi MJ, Langin T and Brygoo Y (1992) FotI, a new family of fungal transposable elements. Mol. Gen. Genet. 232 12-16
Drocourt D, Calmels T, Reynes JP, Baron M and Tiraby G(1990) Cassettes of the *Sireptoalloteichus hindustanus ble* gene for transformation of lower and higher eukaryotes to phleomycin resistance. Nucleic Acids Res. 18 (13), 4009
Eickbush TH (1992). Transposing without ends : the non-LTR retrotransposable elements. New Biol. 4, 430-440
Finnegan DJ (1989) Eukaryotic transposable elements and geneome evolution. Trends Genet. 5, 103-107
Gems D, Johnstone IL and Clutterbuck AJ (1991) An autonomously-replicating plasmid transforms Aspergillus nidulans at high frequency. Gene 98, 61-67
Gierl A (1996) The *En*/*Spm* transposable element in maize. Curr Top Microbiol Immunol. 204, 145-159
Hua-Van A (1998) Caractérisation de la famille d' éléments transposables *Impala* et développement d'un outil de mutagénèse insertionnelle chez le champignon phytopathogène *Fusarium oxysporum*. Thèse de Doctorat. Université Paris 6
Hua-Van A, Héricourt F, Capy P, Daboussi MJ and Langin T (1998) Three highly divergent subfamilies of the *Impala* transposable element coexist in the genome of the fungus *Fusarium oxysporum*. Mol. Gen. Genet. 259, 354-362
Kempken F and Kück (1998) Transposons in filamentous fungi-facts and perspectives. BioEssays 20, 652-659
Kidwell MG (1994) The evolutionary history of the P family of transposable elements. J Hered. 85, 339-346
Kinsey AJ and Helber J (1989) Isolation of a transposable element from *Neurospora crassa*. Proc. Natl. Acad. Sci. USA 86, 1929-1933
Langin T, Daboussi MJ, Gerlingfer C and Brygoo Y (1990) Influence of biological parameters and gene transfer technique on transformation of *Fuarium oxysporum*. Curr. Genet. 17, 313-319
Langin T, Capy P and Daboussi MJ (1995) The transposable element *Impala*, a fungal member of the *Tcl-mariner* superfamily
Lau G and Hamer (1996) Regulatory genes controlling *MPG*1 expression and pathogenicity in the rice blast fungus *Magnaporthe grisea*. Plant Cell, 8, 771-781 Malardier L, Daboussi MJ, Julien J, Roussel F, Scazzocchio C and Brygoo Y (1989) Cloning of the nitrate reductase gene (*niaD*) of *Aspergillus nidulans* and its use for transformation of *Fusarium oxysporum*. Gene 78, 147-156
Okada N and Hamada M (1997) The 3'ends of tRNA-derived SINEs originated from the 3'ends of LINEs : a new example from the bovine genome. J. Mol. Evol. S52-56
Ou SH (1985) Blast. In : C.M. Institute (ed) Rice diseases. CAB International, Kew, UK, 109-201
Pall and Brunelli (1993) Fungal Genet Newsl. 40, 59-63
Prolla TA, Christie DM and Liskay RM (1994) Dual requirement in yeast DNA Mismatch repair for *MLH1* and *PMS1*, two homolgs of the bacterial *mutL* gene. Mol. Cell. Biol 14 (1) 407-415
Punt PJ, Dingemanse MA, Kuyvenhoven A, Soede RD, Pouwels PH and van den Hondel CAMJJ (1990) Functional elements in the promoter region of the Aspergillus nidulans gpdA gene encoding glyceraldehyde-3-phosphate dehydrogenase. Gene 93, 101-109
Punt PJ, Oliver RP, Dingemanse MA, Pouwels PH and van den Hondel CAMJJ (1997) Transformation of Aspergillus based on hygromycin B resistance marker from Escherichia coli. Gene 56, 117-124
Sambrook J, Fritsch EF and Maniatis T (1989) Molecular cloning : a laboratory manual, second edition. Cold Spring Harbor Laboratory Press, New York.
Smit AF and Riggs AD (1996) Tiggers and DNA transposon fossils in the human geneome. Proc. Natl. Acad. Sci. USA 93, 1443-1448
Sweigard JA, Orbach MJ, Valent B and Chumley FG (1990) A miniprep procedure for isolating genomic DNA from *Magnaporthe grisea*. Fungal Genetic Newsletter 37, 4 Sweigard JA, Chumley FG and Valent B (1992) Disruption of a *Magnaporthe grisea* cutinase gene. Mol. Gen. Genet. 232, 183-190
Sweigard JA, Chumley FG, Carroll AM, Farrall L, and Valent B (1997) A series of vectors for fungal transformation. Fungal Genet. Newsl. 44, 52-53
Sweigard JA, Carroll AM, Farrall L, Chumley FG and Valent B (1998) *Magnaporthe grisea* pathogenicity genes obtained through insertional mutagenesis. Mol. Plant-Microbe Interact. 11, 404-412

## Revendications

1. Polynucléotide **caractérisé en ce qu'**il comprend un gène marqueur inactivé par l'insertion d'un transposon *Impala*, ledit gène marqueur, comprenant dans le sens de la transcription une séquence de régulation promotrice fonctionnelle dans *Magnaporthe grisea* liée de manière fonctionnelle à la séquence codante dudit gène marqueur.

2. Polynucléotide selon la revendication 1, **caractérisé en ce que** la séquence de régulation promotrice est une séquence de régulation promotrice d'un gène de *Magnaporthe grisea*, ou d'un autre champignon, en particulier d'un autre champignon filamenteux.

3. Polynucléotide selon l'une des revendications 1 ou 2, **caractérisé en ce que** la séquence de régulation promotrice est constituée par la séquence de régulation promotrice d'un gène de *niaD* ou *gpdA* de champignon.

4. Polynucléotide selon la revendication 3, **caractérisé en ce que** la séquence de régulation promotrice est une séquence de régulation promotrice du gène *niaD d'Aspergillus nidulans* fonctionnelle dans *Magnaporthe grisea*.

5. Polynucléotide selon la revendication 4, **caractérisé en ce que** la séquence de régulation promotrice du gène *niaD d'Aspergillus nidulans* a une taille supérieure à 0,4 kb.

6. Polynucléotide selon l'une des revendications 1 à 5, **caractérisé en ce que** la séquence codante d'un gène marqueur est choisie parmi les séquences codantes d'un gène rapporteur, en particulier GUS ou GFP, les séquences codantes pour un gène de tolérance à un antibiotique ou un herbicide, en particulier les gènes de résistance à l'hygromycine *(hph),* à la phléomycine (*ble*), à l'herbicide bialaphos (*Bar*), ou un gène de résistance aux sulfonylurées.

7. Polynucléotide selon l'une des revendications 1 à 5, **caractérisé en ce que** le gène marqueur est choisie parmi les gènes codant pour des enzymes fonctionnelles dans les champignons, en particulier codant pour une nitrate réductase (*niaD*) ou une nitrilase.

8. Polynucléotide selon la revendication 7, **caractérisé en ce que** le gène marqueur est le gène de la nitrate réductase d'*Aspergillus nidulans*.

9. Polynucléotide selon l'une des revendications 1 à 8, **caractérisé en ce que** le transposon *Impala* est intégré dans la séquence de régulation promotrice du polynucléotide selon l'invention.

10. Polynucléotide selon l'une des revendications 1 à 9, **caractérisé en ce que** le transposon *Impala* comprend un gène marqueur.

11. Polynucléotide selon l'une des revendications 1 à 10, **caractérisé en ce que** le transposon *Impala* est défectif.

12. Méthode pour la préparation de mutants d'insertion de champignons **caractérisée en ce qu'**elle comprend les étapes suivantes:
a) on transforme ledit champignon avec un polynucléotide comprenant un gène marqueur inactivé par l'insertion d'un transposon *Impala* selon l'une des revendications 1-10 dans des conditions permettant l'excision du transposon *Impala* dudit gène marqueur et sa réinsertion dans le génome du champignon ;
b) on identifie les mutants d'insertion exprimant le gène marqueur.

13. Méthode pour la préparation de mutants d'insertion de champignons **caractérisée en ce qu'**elle comprend les étapes suivantes:
a) on transforme ledit champignon avec un polynucléotide comprenant un gène marqueur inactivé par l'insertion d'un transposon *Impala* défectif selon la revendication 11;
b) on mobilise le transposon *Impala* défectif par une transposase dont l'expression est contrôlée dans des conditions permettant l'excision du transposon *Impala* défectif, sa réinsertion et sa stabilisation dans le génome du champignon;
c) on identifie les mutants d'insertion exprimant le gène marqueur.

14. Méthode pour l'identification d'un gène associé à un phénotype détectable chez les champignons **caractérisée en ce qu'**elle comprend les étapes suivantes:
a) on prépare des mutants d'insertion par insertion d'un transposon *Impala* dans le génome desdits champignons selon l'une des méthodes des revendications 12 ou 13;
b) on sélectionne au moins un mutant d'insertion possédant ledit phénotype détectable;
c) on isole le gène dans lequel ou à proximité duquel s'est inséré le transposon *Impala*.

15. Organisme hôte non humain, transformé avec un polynucléotide selon l'une des revendications 1 à 11.

16. Organisme hôte selon la revendication 15 **caractérisé en ce que** l'organisme hôte est un champignon.

17. Champignon dans le génome du quel est intégré un polynucléotide selon l'une des revendications 1 à 11.

18. Champignon selon la revendication 17, **caractérisé en ce que** le gène marqueur est un gène de nitrate réductase de champignon et le champignon est nia-.

## Patentansprüche

1. Polynukleotid, **dadurch gekennzeichnet, daß** es ein durch Insertion eines Impala-Transposons inaktiviertes Markergen umfaßt, wobei dieses Markergen in Transkriptionsrichtung eine in *Magnaporthe grisea* funktionsfähige Promoterregulationssequenz in funktioneller Verknüpfung mit der Codiersequenz dieses Markergens umfaßt.

2. Polynukleotid nach Anspruch 1, **dadurch gekennzeichnet, daß** es sich bei der Promoterregulationssequenz um eine Promoterregulationssequenz eines Gens von *Magnaporthe grisea* oder eines anderen Pilzes, insbesondere eines anderen Fadenpilzes, handelt.

3. Polynukleotid nach Anspruch 1 oder 2, **dadurch gekennzeichnet, daß** die Promoterregulationssequenz aus der Promoterregulationssequenz eines pilzlichen *niaD-* oder *gpdA*-Gens besteht.

4. Polynukleotid nach Anspruch 3, **dadurch gekennzeichnet, daß** es sich bei der Promoterregulationssequenz um eine in *Magnaporthe grisea* funktionsfähige Promoterregulationssequenz des niaD-Gens von *Aspergillus nidulans* handelt.

5. Polynukleotid nach Anspruch 4, **dadurch gekennzeichnet, daß** die Promoterregulationssequenz des *niaD*-Gens von *Aspergillus nidulans* eine Größe von über 0,4 kB aufweist.

6. Polynukleotid nach einem der Ansprüche 1 bis 5, **dadurch gekennzeichnet, daß** die Codiersequenz für ein Markergen aus der Reihe Sequenzen, die für ein Reportergen, insbesondere ein GUS- oder GFP-Reportergen, codieren, Sequenzen, die für ein Gen für Toleranz für ein Antibiotikum oder ein Herbizid, insbesondere Gene für Resistenz gegen Hygromycin (*hph*), Phleomycin (*ble*), das Herbizid Bialaphos (*Bar*), oder ein Gen für Resistenz gegen Sulfonylharnstoffe codieren, stammt.

7. Polynukleotid nach einem der Ansprüche 1 bis 5, **dadurch gekennzeichnet, daß** das Markergen aus der Reihe Gene, die für in Pilzen funktionsfähige Enzyme codieren, insbesondere die für eine Nitratreduktase (*niaD*) oder eine Nitrilase codieren, stammt.

8. Polynukleotid nach Anspruch 7, **dadurch gekennzeichnet, daß** es sich bei dem Markergen um das Gen für die Nitratreduktase von *Aspergillus nidulans* handelt.

9. Polynukleotid nach einem der Ansprüche 1 bis 8, **dadurch gekennzeichnet, daß** das Impala-Transposon in die Promoterregulationssequenz des erfindungsgemäßen Nukleotids integriert ist.

10. Polynukleotid nach einem der Ansprüche 1 bis 9, **dadurch gekennzeichnet, daß** das Impala-Transposon ein Markergen umfaßt.

11. Polynukleotid nach einem der Ansprüche 1 bis 10, **dadurch gekennzeichnet, daß** das Impala-Transposon defizient ist.

12. Verfahren zur Herstellung von Insertionsmutanten von Pilzen, **dadurch gekennzeichnet, daß** es die folgenden Schritte umfaßt:
a) man transformiert den Pilz mit einem Polynukleotid nach einem der Ansprüche 1-10, das ein durch Insertion eines Impala-Transposons inaktiviertes Markergen umfaßt, unter Bedingungen, die das Herausschneiden des Impala-Transposons von dem Markergen und seine erneute Insertion in das Genom des Pilzes gestatten;
b) man identifiziert die Insertionsmutanten, die das Markergen exprimieren.

13. Verfahren zur Herstellung von Insertionsmutanten von Pilzen, **dadurch gekennzeichnet, daß** es die folgenden Schritte umfaßt:
a) man transformiert den Pilz mit einem Polynukleotid nach Anspruch 11, das ein durch Insertion eines Impala-Transposons inaktiviertes Markergen umfaßt
b) man mobilisiert das defiziente *Impala*-Transposon mit einer Transposase, deren Expression unter Bedingungen kontrolliert wird, die das Herausschneiden des defizienten Impala-Transposons, seine erneute Insertion in das Genom des Pilzes und seine Stabilisierung in dem Genom des Pilzes gestatten;
c) man identifiziert die Insertionsmutanten, die das Markergen exprimieren.

14. Verfahren zur Identifikation eines Gens, das mit einem in Pilzen nachweisbaren Phänotyp assoziiert ist, **dadurch gekennzeichnet, daß** es die folgenden Schritte umfaßt:
a) man stellt durch Insertion eines *Impala*-Transposons in das Genom dieser Pilze nach einem der Verfahren der Ansprüche 12 oder 13 Insertionsmutanten her;
b) man selektiert mindestens eine Insertionsmutante, die den nachweisbaren Phänotyp aufweist;
c) man isoliert das Gen, in das bzw. in dessen Nähe sich das Impala-Transposon insertiert hat.

15. Nichtmenschlicher Wirtsorganismus, transformiert mit einem Polynukleotid nach einem der Ansprüche 1 bis 11.

16. Wirtsorganismus nach Anspruch 15, **dadurch gekennzeichnet, daß** es sich bei dem Wirtsorganismus um einen Pilz handelt.

17. Pilz, in dessen Genom ein Polynukleotid nach einem der Ansprüche 1 bis 11 integriert ist.

18. Pilz nach Anspruch 17, **dadurch gekennzeichnet, daß** es sich bei dem Markergen um ein pilzliches Nitratreduktasegen handelt und daß der Pilz niaist.

## Claims

1. Polynucleotide, **characterized in that** it comprises a marker gene which is inactivated by the insertion of an *Impala* transposon, said marker gene comprising, in the direction of transcription, a promoter regulatory sequence which is functional in *Magnaporthe grisea* and which is functionally linked to the coding sequence of said marker gene.

2. Polynucleotide according to Claim 1, **characterized in that** the promoter regulatory sequence is a promoter regulatory sequence of a gene from *Magnaporthe grisea,* or from another fungus, in particular from a filamentous fungus.

3. Polynucleotide according to either of Claims 1 and 2, **characterized in that** the promoter regulatory sequence consists of the promoter regulatory sequence of a fungal *niaD* or *gpdA* gene.

4. Polynucleotide according to Claim 3, **characterized in that** the promoter regulatory sequence is a promoter regulatory sequence of the *niaD* gene from *Aspergillus nidulans*, which is functional in *Magnaporthe grisea.*

5. Polynucleotide according to Claim 4, **characterized in that** the promoter regulatory sequence of the *niaD* gene from *Aspergillus nidulans* is more than 0.4 kb long.

6. Polynucleotide according to one of Claims 1 to 5, **characterized in that** the coding sequence of a marker gene is chosen from the coding sequences of a reporter gene, in particular GUS or GFP, the coding sequences for a gene for tolerance to an antibiotic or herbicide, in particular the genes for resistance to hygromycin (hph), to phleomycin (*ble*) or to the herbicide bialaphos *(Bar),* or a gene for resistance to sulphonylureas.

7. Polynucleotide according to one of Claims 1 to 5, **characterized in that** the marker gene is chosen from the genes encoding enzymes which are functional in fungi, in particular encoding a nitrate reductase (*niaD*) or a nitrilase.

8. Polynucleotide according to Claim 7, **characterized in that** the marker gene is the nitrate reductase gene from *Aspergillus nidulans*.

9. Polynucleotide according to one of Claims 1 to 8, **characterized in that** the *Impala* transposon is integrated into the promoter regulatory sequence of the polynucleotide according to the invention.

10. Polynucleotide according to one of Claims 1 to 9, **characterized in that** the *Impala* transposon comprises a marker gene.

11. Polynucleotide according to one of Claims 1 to 10, **characterized in that** the *Impala* transposon is defective.

12. Method for preparing insertion mutants of fungi, **characterized in that** it comprises the following steps:
a) said fungus is transformed with a polynucleotide comprising a marker gene which has been inactivated by the insertion of an *Impala* transposon according to one of Claims 1 to 10, under conditions which allow the excision of the *Impala* transposon of said marker gene and its reinsertion into the genome of the fungus;
b) the insertion mutants expressing the marker gene are identified.

13. Method for preparing insertion mutants of fungi, **characterized in that** it comprises the following steps:
a) said fungus is transformed with a polynucleotide comprising a marker gene which has been inactivated by the insertion of a defective *Impala* transposon according to Claim 11;
b) the defective *Impala* transposon is mobilized using a transposase, the expression of which is controlled, under conditions which allow the excision of the defective *Impala* transposon, its reinsertion and its stabilization in the genome of the fungus;
c) the insertion mutants expressing the marker gene are identified.

14. Method for identifying a gene associated with a detectable phenotype in fungi, **characterized in that** it comprises the following steps:
a) insertion mutants are prepared by inserting an *Impala* transposon into the genome of said fungi according to one of the methods of Claims 12 or 13;
b) at least one insertion mutant with said detectable phenotype is selected;
c) the gene into which, or close to which, the *Impala* transposon has inserted is isolated.

15. Non human host organism transformed with a polynucleotide according to one of Claims 1 to 11.

16. Host organism according to Claim 15, **characterized in that** the host organism is a fungus.

17. Fungus into the genome of which is integrated a polynucleotide according to one of Claims 1 to 11.

18. Fungus according to Claim 17, **characterized in that** the marker gene is a fungal nitrate reductase gene and the fungus is nia-.
